# EUROPEAN PATENT SPECIFICATION

(11) **EP 0 691 868 B1**
(45) Date of publication and mention of the grant of the patent: **12.06.2002**
(21) Application number: 94912808.6
(22) Date of filing: 18.03.1994
(51) Int. Cl.: A61M 25/02

(54) **CATHETER ANCHORING SYSTEM**
KATHETERBEFESTIGUNGSSYSTEM
SYSTEME D'ANCRAGE DE CATHETER

(30) Priority: 19.03.1993 US 34340; 15.09.1993 US 121942
(43) Date of publication of application: 17.01.1996
(60) Divisional application: 01204338.6
(73) Proprietor: Venetec International, Inc., Mission Viejo, California 92691 (US)
(72) Inventor: Bierman, Steven, Del Mar, CA 92014 (US)
(74) Representative: W.P. Thompson & Co.
(86) International application number: US9402944
(87) International publication number: WO9421319

(56) References cited:
- US-A- 4 224 937
- US-A- 4 898 587
- US-A- 5 192 273

## Description

The present invention relates to a catheter anchoring system.

More particularly the present invention relates to a catheter anchoring system for use in a percutaneous catheterization system, which can securely interconnect an indwelling catheter with a tubing and securely anchor such an interconnection to a patient's skin.

Medical treatment of patients commonly involves the use of percutaneously inserted catheters to direct fluids directly into the bloodstream, a specific organ or an internal location of the patient, or to monitor vital functions of the patient. For instance, intra-arteriosus catheters are commonly used to direct fluids and/or medication directly into the bloodstream of the patient. Epidural catheters are commonly used to direct anesthesia into an epidural space to anesthetize a specific location of the patient. Intervascular catheters are commonly used to monitor arterial blood pressure.

The fluid (e.g., parenteral liquid, medication or anesthesia) typically drains from a container positioned above the patient. The fluid flows through tubing and into an indwelling catheter. The catheter and fluid tubing are commonly removably attached by a conventional lure-type connector, such as the type described in U.S. Patent No. 4,224, 937.

In common practice, a health care provider, such as, for example, a nurse or doctor (for ease of description, as used herein the term "nurse" will refer to health care providers generally and will not be restrictive in meaning), uses adhesive or surgical tape to maintain the catheter in place on the skin of the patient. The connection between the tubing and the catheter is likewise maintained by use of tape.

The nurse may also form a safety loop in the tubing so that any tension applied to the tubing does not directly pass to the catheter cannula, but rather is absorbed by the slack of the safety loop. The nurse typically loosely tapes the loop to the skin of the patient.

This entire taping procedure takes several minutes of the valuable time of the health care provider. Furthermore, nurses commonly remove their gloves when taping because most nurse find such taping procedures difficult and cumbersome when wearing gloves.

The catheterization process often requires frequent disconnection between the catheter and the fluid supply tube. For instance, intravenous catheterization is frequently maintained for several days, depending upon the condition of the patient. The catheter tubing is generally replaced every 24 to 48 hours in order to maintain the sterility of the fluid and the free-flow of the fluid through the tubing. A nurse must thus frequently change the tubing and retape the connection. Moreover, the tape, which secures the catheter to the skin of the patient, often covers the cannula insertion point. The nurse must remove the tape to inspect the insertion point for inflammation or infection, and must then repeat the above-described taping procedure.

A great deal of valuable time is thus used in applying significant amounts of surgical tape to indwelling catheters. The frequent application and removal of surgical tape also commonly results in the excoriation of the skin of the patient in the area of the insertion.

A number of catheterization systems have recently been developed which improve the stabilization of the catheter system and obviate the need for frequent application and removal of surgical tape. One such system is disclosed by U.S. Patent No. 5,192,273 issued to the present Applicant.

The '273 patent discloses an adaptor which interconnects the catheter with a fluid supply tubing. The adaptor snaps into a base attached to the patient's skin by an adhesive pad. Specifically, a nurse presses the adaptor between upstanding legs of the base. Detents on the legs slide into corresponding annular grooves in the adapter to hold the adapter to the base.

Although the base holds the adaptor securely in place, a nurse may have difficulty positioning and aligning the annular grooves of the adaptor with the detents on the base. Exigent circumstances may further exacerbate the difficulties associated with properly positioning the adaptor onto the base. Some nurses and other health care providers may also have trouble determining how to engage the catheter adaptor with the base.

Another catheterization system is described in U.S. Patent No. 4,898,587. The '587 patent discloses a device for holding a medical tube to a body. This device includes a base plate for adhering the device to a part of a body, cushioned straps which secure the medical tube to the base plate, and a cover which overlies the base plate and a portion of the tube.

The aim of the present invention is to provide a catheter anchoring system having an adaptor retainer which is not position or technique sensitive. That is, the nurse simply locates the catheter adaptor generally above the retainer, and presses the adaptor into the retainer. Engagement requires only course alignment of the adaptor with the retainer.

According to one aspect of the present invention there is provided an anchoring system comprising:
a catheter adaptor defined between a distal end configured to permit engagement with tubing or the proximal hub of a catheter or lure-type connector and a proximal end configured to couple to a distal end of a tube, the catheter adaptor having a generally tubular body;
a retainer having a longitudinal channel configured to receive the catheter adaptor tubular body in a snap fit manner;
a flexible anchor pad including an adhesive bottom surface, the anchor pad supporting the retainer; wherein
the catheter adaptor further comprises a radially extending member disposed along the length of the tubular body, which radially extending member projects from an exterior surface of the tubular body; wherein
the retainer comprises a pair of opposed longitudinally extending walls, defining between them a longitudinal channel having a generally circular cross section truncated at an upper end to form a generally U-shaped channel having an upper opening and a series of slots, sized to receive and capture said radially extending member, the series of slots being formed in each of the walls, each slot in one wall being in alignment with a corresponding slot in the other wall.

According to another aspect of the present invention there is provided a method of anchoring an inter connection between a catheter adaptor and a tube comprising:
a. providing a catheter adaptor defined between a distal end configured to permit engagement with tubing or the proximal hub of a catheter or lure-type connector and a proximal end configured to couple to a distal end of the tube, the catheter adaptor having a generally tubular body;
b. providing a retainer having a longitudinal channel configured to receive in a snap fit manner the catheter adaptor tubular body;
c. providing a flexible anchor pad including an adhesive bottom surface (18); the anchor pad supporting the retainer; wherein the catheter adaptor further comprises a radially extending member disposed along the length of the tubular body, which radially extending member projects from an exterior surface of the tubular body; and
   the retainer comprises a pair of opposed longitudinally extending walls defining between them a longitudinal channel having a generally circular cross section truncated at an upper end to form a generally U-shaped channel having an upper opening and a series of slots sized to receive and capture said radially extending member, the series of slots being formed in each of the walls, each slot in one wall being in alignment with a corresponding slot in the other walls;
d. positioning the catheter adaptor radially extending portion above the series of slots; and
e. inserting the catheter adaptor tubular body in to the longitudinal channel and the catheter adaptor radially extending portion in one of the series of slots.

The present invention will now be further described, by way of example, with reference to the accompanying drawings, in which:-
Figure 1 is a perspective view of a catheter anchoring system in accordance with a preferred embodiment of the present invention, mounted on the back of a patient's hand;
Figure 2 is a top plan view of the catheter anchoring system of Figure 1;
Figure 3 is a top plan view of a retainer of the catheter anchoring system of Figure 2;
Figure 4a is a front elevation view of the retainer of Figure 3;
Figure 4b is a rear elevational view of the retainer of Figure 3;
Figure 5 is a side elevational view of the retainer of Figure 3;
Figure 6 is a top plan view of a catheter anchoring system in accordance with another preferred embodiment of the present invention;
Figure 7a is a front elevational view of a retainer and rail assembly of the catheter anchoring system of Figure 6;
Figure 7b is a side elevational view of the retainer and rail assembly of Figure 6;
Figure 8 is a cross-sectional view of the retainer and rail assembly taken along line 8-8 of Figure 7a;
Figure 9 is a top plan view of a catheter anchoring system in accordance with an additional preferred embodiment of the present invention;
Figure 10 is a side elevational view of an S-clip of the catheter anchoring system of Figure 9 taken along line 10-10;
Figure 11 is a top perspective view of a catheter anchoring system in accordance with an additional preferred embodiment of the present invention;
Figure 12 is a top plan view of the catheter anchoring system of Figure 11 illustrating an adaptor held by a retainer;
Figure 13a is a side elevational view of the retainer of Figure 12;
Figure 13b is a top plan view of the retainer of Figure 12; and
Figure 14 is a perspective view of an alternative embodiment of a catheter adaptor which may be used with the anchoring system of Figure 11.

### Detail Description of Preferred Embodiments

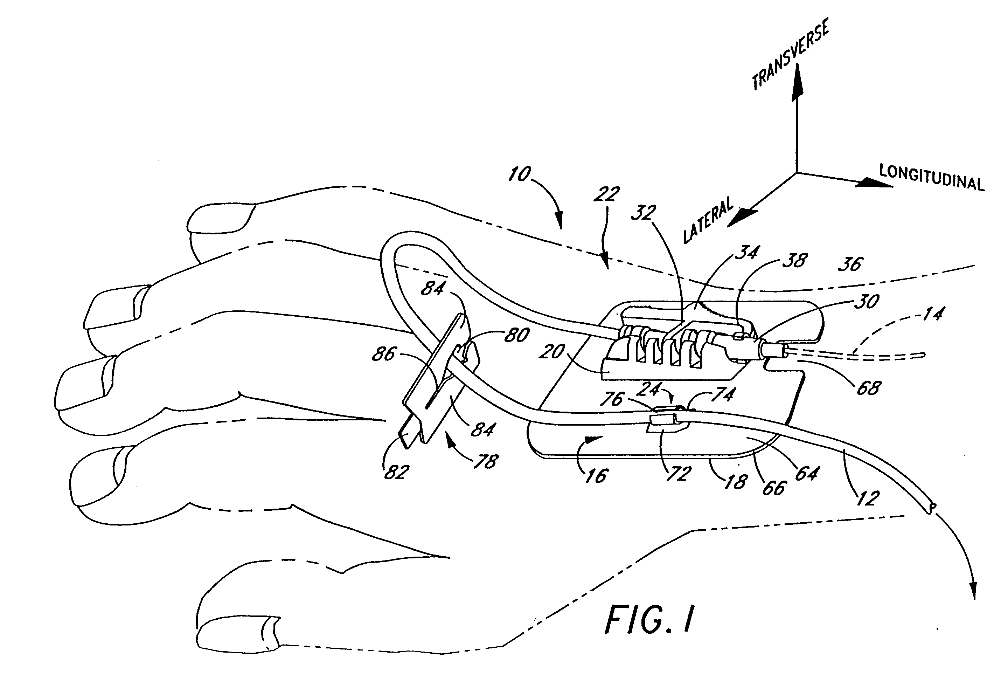

Figure 1 illustrates in perspective view a catheter anchoring system 10 in accordance with the present invention. The anchoring system 10 securely connects a tube 12 (e.g., a fluid supply tube) to an indwelling catheter 14 and maintains the catheter 14 in the desired indwelling position. The anchoring system 10 is designed for rapid attachment to the catheter 14 and to the patient, without requiring precise alignment or positioning of the components of the anchoring system 10.

Moreover, sturdy anchoring of the catheterization system is achieved without the use of surgical tape. For most catheterization, the anchoring system is attached to the patient only once. Although the fluid supply tubing 12 may be replaced every 24 to 48 hours for intravenous catheterization, the components of the anchoring system 10 attached to the patient remains in place. Thus, surgical tape need not be applied and removed from the patient's skin on multiple occasions.

The catheter anchoring system 10 principally comprises a flexible pad 16 having an adhesive bottom side 18 which attaches to the skin of a patient when used. The pad 16 supports a retainer 20. The retainer 20 is configured to receive and secure in place a catheter adaptor 22 which interconnects the hub 30 of an indwelling catheter 14 and the fluid supply tube 12 connected to a fluid supply container (not shown). The container maintains the fluid to be dispensed to the patient which is fed either by gravity or by pressure. A clamp (not shown) may be used to regulate the fluid flow through the tubing 12. The pad 16 may also support a tubing clip 24 which is used to retain a portion of tubing 12.

Although Figure 1 illustrates the catheter anchoring system located on the back of a patient's hand (illustrated in phantom lines), it is contemplated that the present invention may be used for catheterization in other locations on the patient's body. For instance, the anchoring system may be used on the medial side of the wrist in connection with a radial artery. The anchoring system 10 may also be used for epidural catheterization, as discussed in detail below, and thus located on the posterior of the patient's torso.

Figure 1 illustrates a longitudinal axis, a transverse axis and a lateral axis in relation to the catheter anchoring system 10 to facilitate the following description. Additionally, as used herein, "the longitudinal direction" refers to a direction substantially parallel to the longitudinal axis. "The lateral direction" and "the transverse direction" are in reference to the lateral axis and transverse axis, respectively. Also, "proximal" and "distal" are in reference to the proximity of the fluid supply container attached to the tube 12 (see Figure 1). The individual components of the catheter anchoring system 10 will now be described in detail.

### Catheter Adaptor

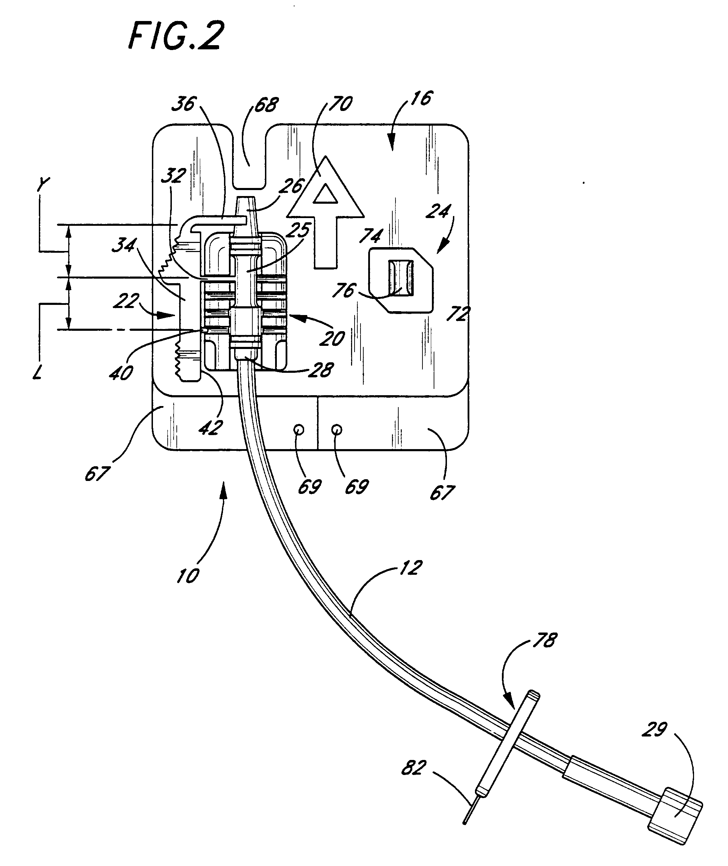

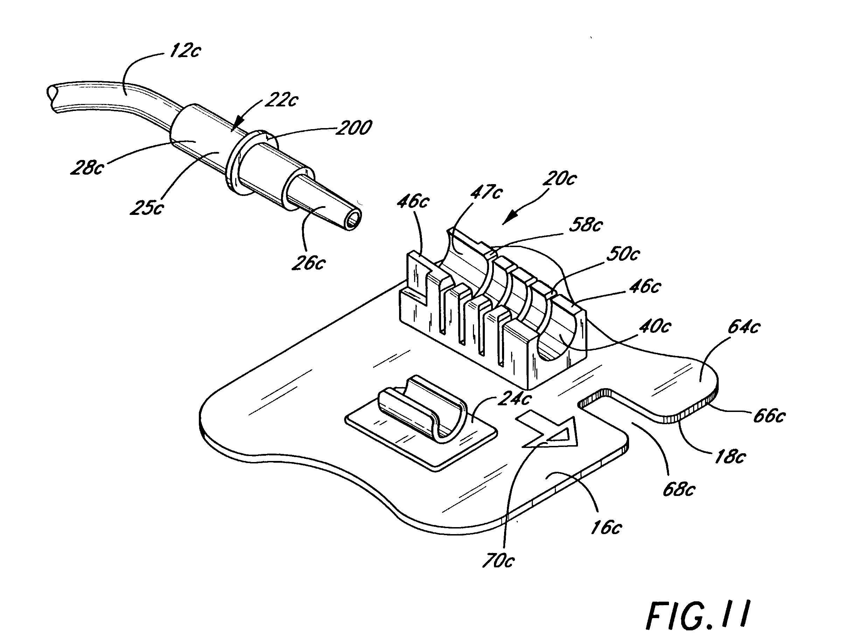

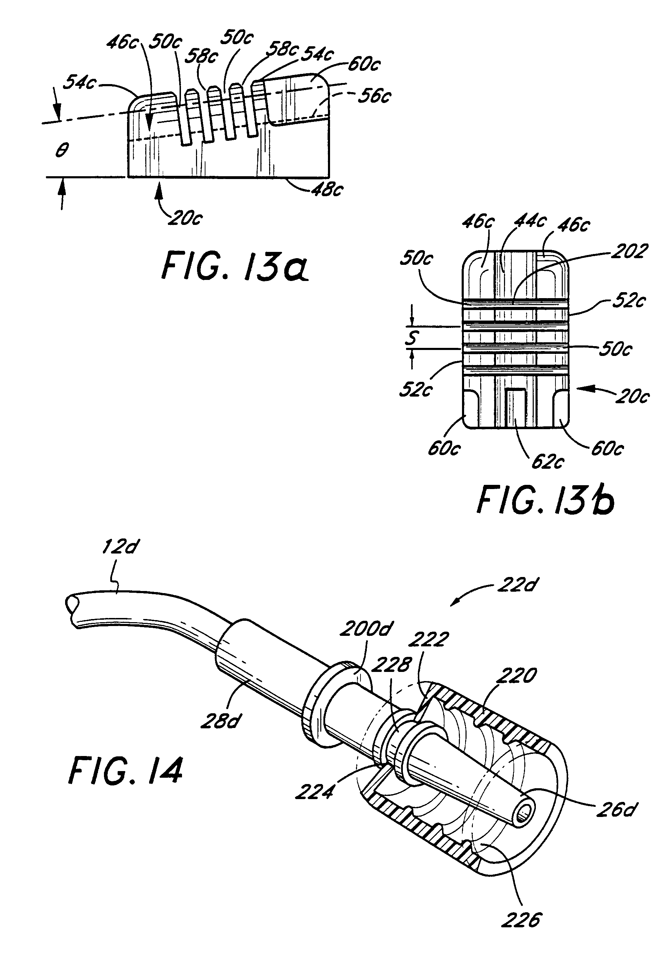

Figure 1 illustrates the catheter adaptor 22 interconnected to a catheter 14. Figure 2 illustrates the catheter adaptor 22 disconnected from catheter 14. Although these figures illustrate the adaptor 22 as the type disclosed in U.S. Patent No. 5,193,273, it is contemplated that other types of adaptors can be used as well with the present catheter anchoring system 10. For instance, the catheter adaptor 22 could be a lure-type adaptor, such as the type illustrated by Figure 11 and described below, or a lure-lock type catheter adaptor 22, such as the type illustrated by Figure 14 and described below. It is contemplated that those skilled in the art could readily select the type of catheter adaptor 22 to be used with the present catheter anchoring system 10 depending on the particular application (e.g., venous, arterial, epidural, etc.) of the anchoring system 10.

As best seen in Figure 2, the adaptor 22 comprises a tubular body 25 defined between a distal end 26 and a proximal end 28. The proximal end 28 is adapted to receive a distal end of the tube 12. In an exemplary embodiment, at least a portion of the fluid supply tube is permanently attached to the body proximal end 28. As shown in Figure 2, the proximal end of the tubing may then include a standard lure-type connector 29 to connect into a fluid supply line 12.

The distal end 26 is configured to engage the proximal hub 30 of the catheter 14 (see Figure 1) or any lure-type connector. Although Figure 2 illustrates the distal end of the adaptor 22 as having a frusto-conical shape configured to engage a standard lure-type catheter hub 30, it is contemplated that the distal end 26 could be configured as well to engage other types of catheter connectors, such as, for example, a Toughy-Bourst adaptor.

A. support arm 32 extends outwardly from the tubular body 25 in cantilever fashion. The support arm 32 supports, on a radially outer end of the arm 32, a clip support element (not shown) that extends generally parallel to and is spaced from a longitudinal axis of the tubular body 25.

Figure 2 further illustrates a clip 34 of the catheter adaptor. The clip 34 attaches to and slides over the clip support element in the longitudinal direction. The clip 34 includes a distal latch 36 which has a generally forked shape to engage a outer surface of the catheter hub 30 distal end of a hub collar 38 (see Figure 1) to securely attach the adaptor 22 to the catheter hub 30.

Interengaging structure (not shown) between the clip support element and the clip 34 permits the clip 34 to slide in the proximal direction, but prevents the clip 34 from sliding in the distal direction. The interengaging element desirably comprises a series of ratchet teeth (not shown) disposed on the upper surface of the clip support element and a pawl (not shown) connected to the clip 34. The pawl extends from the clip 34 in a cantilever fashion and engages the ratchet teeth to prevent distal movement of the clip, as discussed in detail in U.S. Patent No. 5,193,273.

The tubular body 25, the support arm 32 and the clip support element are preferably integrally formed of molded plastic, such as, for example, a clear polycarbonate, so as to be generally stiff, but somewhat flexible. The support arm 32 desirably has enough elasticity to bend. Depressing the proximal end of the clip 34 towards the tubular body 25 moves the latch 36 of the clip 34 away from the tubular body 25. In this manner, the clip 34 pivots about the tubular body 25.

With reference again to Figure 2, the clip support element desirably comprises a protuberance 40 positioned on an inner surface 42 of the clip support element, proximate to the proximal end of the clip 34. The protuberance is spaced from the support arm by a distance L. The protuberance 40 prevents the clip 34 from pivoting when secured by the retainer 20, as discussed below in detail. The protuberance 40 also limits the degree of deflection of the support arm 32 to reduce fatigue, as fully explained in U.S. Patent No. 5,193,273,

### Retainer for Catheter Adaptor

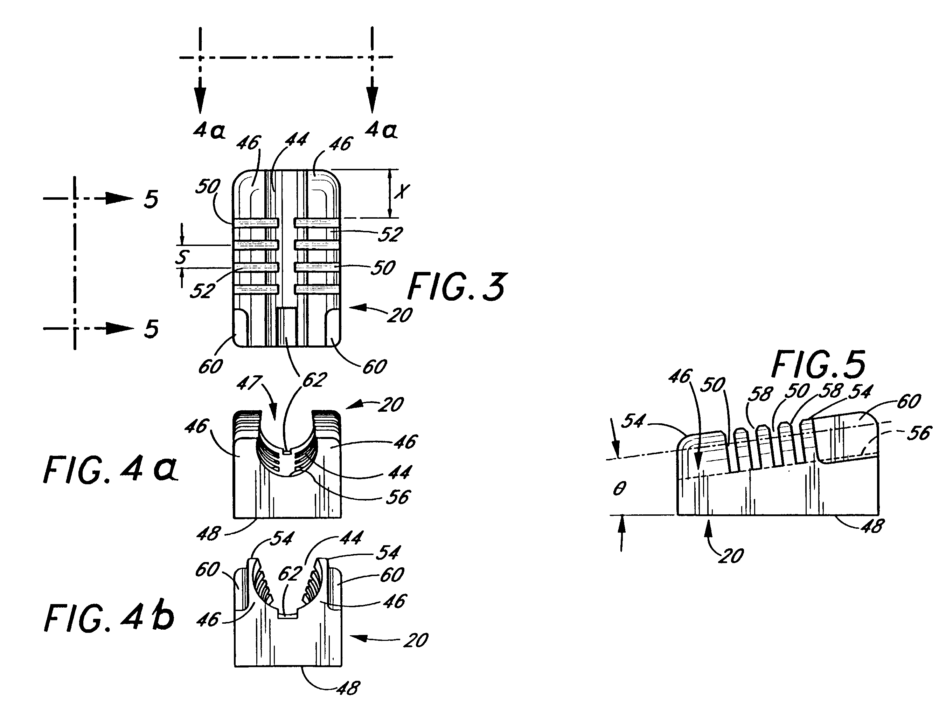

Figures 3 through 5 illustrate the retainer 20. The retainer 20 has a generally parallelepiped shape defining a central channel 44 interposed between a pair of opposing longitudinal walls 46. The central channel 44 extends through the retainer 20 along an axis which is generally parallel to the longitudinal axis of the retainer.

As best seen in Figure 4, the central channel 44 has a generally circular cross-sectional shape which is truncated at its upper end to form a generally U-shaped channel having an upper opening 47. The central channel 44 has a diameter sized to receive the tubular body 25 of the catheter adaptor 22. In a preferred embodiment, the diameter of the central channel 44 generally matches that of the tubular body 25 or is slightly larger.

In cross-section, the central channel 44 extends through an arc greater than 180° about the channel axis such that the transverse length of the opening 47 is less than the diameter of the central channel 44. In an exemplary embodiment, the central channel 44 extends through an arc of about 200° about the channel axis.

Figure 5 illustrates the channel axis which is desirably skewed relative to a base surface 48 of the retainer 20. An incident angle θ formed between the base surface 48 and the channel axis is less than 45°. The incident angle θ desirably ranges between 0° and 30°. In an exemplary embodiment for intravenous use, the angle θ preferably equals approximately 7°. In another exemplary embodiment for arterial use, the incident angle θ preferably equals about 22°. In a further exemplary embodiment, for peripherally inserted central catheters (PICC), the incident angle θ preferably equals 0°.

The longitudinal walls 46 are substantially identical. Each wall 46 has a thickness measured in the lateral direction less than the length of the support arm 32. The wall 46 is thus interposed between the tubular body 25 and the clip 34 when the tubular body 25 is inserted into the central channel 44. The length of each wall 46, measured in the longitudinal direction, is preferably coextensive with the length of the retainer 20.

Each wall 46 comprises a uniform series of slots 50. The series comprises at least two (2) slots 50, and not more than twenty (20) slots 50. More preferably, the series comprises less than seven (7) slots 50. In an exemplary embodiment, as illustrated in the figures of the application, the series comprises four (4) slots 50.

Each slot 50 is sized to receive the support arm 32 of the catheter adaptor 22 to prevent longitudinal displacement of the adaptor 22, as discussed in detail below. Each slot 50 desirably has a rectangular shape. As aeen in Figure 3, the slots 50 extend from an exterior surface 52 through the wall 44, and open into the central channel 44. The width of each slot 50 (measured longitudinally) is desirably slightly greater than the width of the support arm 32, measured in the longitudinal direction to receive the support arm 32, as discussed below.

As illustrated by Figure 5, each slot 50 has a height as measured in the transverse direction between an upper edge 54 of the longitudinal wall 46 and the bottom 56 of the central channel 44. The height of the slot 50 desirably equals approximately the width of the support arm 32 such that the support arm 32 does not protrude from the retainer 20 in the transverse direction.

The spacing S between the slots 50, on center, desirably equals about half the distance L (see Figure 2) between the support arm 32 and the protuberance 40 of the catheter adaptor 22.

As Figure 3 illustrates, a distance X between the most distal slot 50 and the distal end of the retainer 20 is less than the longitudinal distance Y (see Figure 2) between the support arm 32 and the latch 36 positioned in its most proximal position. This spacing enables the support arm 32 to rest in the most distal slot 50 with the latch 36 retaining a catheter hub 30 distal of the retainer distal end.

Figure 5 illustrates the upper edge 50 of the longitudinal wall 46 which comprises a series of chamfers 58, each of which slopes into a slot 50. That is, the portion of upper edge 50 of the longitudinal wall 46 which surrounds a slot 50 includes a pair of chamfers 58, with one chamfer 58 located on either side of the slot 50. The chamfers 58 slope downward toward the slot 50 to facilitate the insertion of the support arm 32 of the catheter adaptor 22 into the slot 50, as discussed below.

As shown by Figures 3 and 5, each longitudinal wall 46 further comprises a relief 60 disposed on the proximal end of the retainer 20. The relief 60 is sized to receive the protuberance 40 of the adaptor 22. The depth of the relief 60 measured in the lateral direction desirably is slightly greater than the height of the protuberance 40 (i.e., the distance by which the protuberance protrudes from the inner surface 42).

The relief 60 is spaced in the longitudinal direction from the most proximal slot 50 by a distance approximately equal to the spacing S between the slots 50. Thus, the protuberance 40 rests in the relief 60 with the support arm 32 positioned in either of the two most proximal slots 50, as discussed in detail below.

Figures 3 and 4 illustrate a key-way groove 62 of the retainer 20. The key-way groove 62 facilitates the removal of the catheter adaptor 22 from the retainer 20, as discussed below in detail. The key-way groove 62 lies at the proximal end of the retainer 20. The key-way groove 62 extends into the retainer 20, and toward the retainer base surface 48 from the bottom surface 56 of the central channel 44. The key-way groove 62 has a transverse width less than the diameter of the central channel 44, and more preferably has a width approximately equal to two-thirds, the diameter of the central channel 44. The longitudinal length of the key-way groove 62 desirably equals approximately the longitudinal length of the recesses 60 in the longitudinal walls 46.

The retainer 20 is made of relatively stiff plastic material (e.g., polycarbonate), but is somewhat flexible such that the adaptor 22 forces the upper edges 54 of the longitudinal walls 46 outwardly when a nurse presses the adaptor 24 into the central channel 44 of the retainer 20. When the adaptor 22 sits in the central channel 44, the upper edges 54 of the walls 46 snap inwardly to their original position to securely hold the adaptor 22 within the retainer 20.

An adhesive attaches the retainer 20 to base pad 16. Alternatively, the retainer 20 may be attached to the base pad 16 by like means (e.g., embedding or otherwise weaving the retainer 20 into the base pad 16) as well.

### Base Pad

As illustrated by Figure 1, the flexible base pad 16 comprises a laminate structure comprising an upper paper or other woven or non-woven cloth layer 64, an inner cellulose foam layer 66, and the bottom adhesive layer 18. Alternative, the flexible base pad 16 may comprise an adhesive bottom layer and an upper cellulose foam layer. An upper surface of the foam layer is roughened by corona treating the foam with a low electric charge, as known in the art. The roughened or porous upper surface of the base pad 16 improves epoxy adhesion when attaching the retainer 20 to the pad 16.

A removable paper or plastic backing (not shown) desirably covers the bottom adhesive layer 18 before use. The backing preferably resists tearing and is divided into a plurality of pieces to ease attachment of the pad 16 to the patient's skin, as explained below. Desirably, the backing is split along the center line of the flexible base pad 16 in order to expose only half of the adhesive bottom surface 18 at one time. The backing also advantageously extends beyond at least one edge of the base pad 16 to ease removal of the backing from the adhesive layer 18.

As seen in Figure 2, one or more tabs 67 may be attached to a portion of the backing which extends beyond the flexible base pad 16. In an exemplary embodiment, the tabs 67 have the same laminate structure as the flexible base pad 16. The tab 67 may also include indicia 69 in the form of dots, words, figures or the like to indicate the placement of fingers when removing the backing from the base pad 16.

A nurse grips the tab 67, preferably at the location of the indicia 69, and peels the backing off one half of the bottom adhesive layer 18. The nurse then places the bottom layer 18 against the patient's skin to adhere the base pad 16 to the patient. Light pressure over the upper layer 64 assures good adhesion between the base pad 16 and the patient's skin. The base pad 16, due to its flexibility, conforms to the contours of the topical surface to which the base pad 16 adheres. The nurse then repeats this procedure for the other half of the pad 16. Alternatively, the nurse may completely remove the backing from the pad 16 before attaching the pad 16 to the patient's skin.

The base pad 16 desirably comprises a notch 68 positioned distal of the location of the retainer 20 on the pad 16 and adjacent to the point of insertion of the catheter cannula. The notch 68 is sized to permit visual inspection of the catheterized site.

As seen in Figure 2, the base pad 16 desirably comprises indicia 70 in the form of an arrow which indicates the proper orientation of the base pad 16 in reference to catheterized site. Although the figures illustrate the indicia in the form of an arrow, it is contemplated that other forms of indicia, such as, for example, words or other graphics, could be used as well. In proper use, as illustrated in Figure 1, the indicia 70 should point in the proximal direction, towards the indwelling catheter 14. or otherwise indicate the proper location of the pad 16 with reference to the indwelling catheter 14.

In an exemplary embodiment, the laminate structure of the base pad is preferably formed by rolling a paper tape, such as a micro-porous rayon tape, available commercially as MICROPORE tape from 3M (Item No. 1530), over a medical grade polyvinyl chloride foam tape, such as that available commercially from 3M (Item No. 9777L). The foam tape preferably includes the bottom liner or backing. The base pad 16 and the taps 67 are then stamped out of the laminated sheet of foam and paper. The backing between the taps and the base pad, however, is desirably not severed such that the tabs 67 remain attached to the backing covering the adhesive section 18 of the base pad 16. The backing is then cut into two pieces along the center line of the pad 16 and between the tabs 67.

### Tube Clip

Figures 1 and 2 illustrate the tube clip 24. The clip 24 secures the fluid supply tube 12 to form a safety loop, as known in the art.

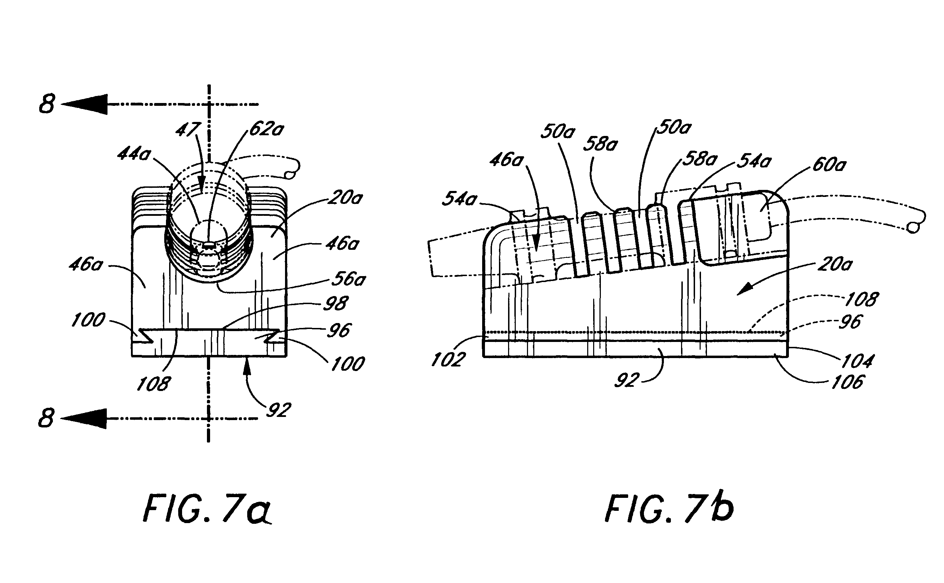

The tube clip has a plate-like base 72 adhered to or embedded in the base pad 16. The tube clip 24 may be located on the base pad 16 on either side of the retainer 20 to accommodate left hand or right hand mounting. As illustrated in Figure 6, the anchoring system 10 may further include a second tube clip 24 located on the other side of the retainer 20 from the first tube clip 24.

The clip 24 defines a channel 74 having a generally circular cross-sectional configuration truncated to form an upper orifice 76. The diameter of the channel 74 is desirably slightly less than that of the fluid supply tube 12 so as to ensure a secure interconnection. The channel 74 receives a portion of the fluid supply tube 12 through the orifice 76 upon application of gentle pressure or by pulling the tubing 12 across and through the orifice 76 of the tube clip 24, as explained below. The clip 24 surrounds a substantial portion of the tubing 12 with the tubing 12 positioned within the channel 74.

As seen in Figure 2, the upper edge of the channel includes tapered ends 77 at the proximal and distal ends of the clip 24. Each tapered end 77 forms a smooth transition between the side edge of the channel 74 and the upper edge, and tapers in lateral width from the side edge toward the center of the tube clip 24. The tapered ends 77 help guide the fluid supply tube 12 into the channel 74 when a nurse pulls the tube across the clip 24. Thus, the nurse does not have to pinch the tube 12 to insert it into the clip 24. Also, the nurse's gloves do not get stuck in the clip 24 when inserting the tube 12, as is typically the case where the nurse is required to pinch the tube 12 to insert it into the clip 24.

### Slide Clamp

As illustrated in Figures 1 and 2, the catheter anchoring system 10 desirably additionally includes a slide clamp 78 to regulating fluid flow through the tubing, as known in the art. The clamp 78, at one end, includes an aperture 80 which receives the fluid supply tube 12, and, at the opposite end, includes a tab 82. The clamp 78 has a generally forked shape formed by a pair of prongs 84 which defines the aperture 80. The tube 12 snaps between the prongs 84 and into the aperture 80, which has a diameter slightly larger than the fluid supply tube 12.

The prongs 84 converge together in the direction towards the tab 82 to form a tapering slot 86 which opens into the aperture 80. The prongs 84 pinch the tube 12 closed with the tube 12 positioned in the slot 86 so as to block fluid flow therethrough. The clamp 78, however, slides over the tube 12 with the tube 12 positioned through the aperture 80.

The tab 82 desirably has a rectangular shape which generally corresponds the to shape of the key-way groove 62 of the retainer 20. The tab 82 preferably has a thickness greater than that of the distal end of key-way groove 62, measured in the transverse direction, so as to pry the adaptor 22 from the retainer 20. As explained in detail below, the tab 82 may be used to remove the catheter adaptor 22 from the retainer 20.

### Retainer Location Adjustment Mechanism

Figure 6 through 8 illustrate a catheter anchoring system 10a in accordance with another preferred embodiment of the present invention. Where appropriate, like numbers with an "a" suffix have been used to indicate like parts of the two embodiments for ease of understanding.

The catheter anchoring system 10a is substantially identical to the above-described anchoring system 10, with the addition of a retainer location adjustment mechanism 90.

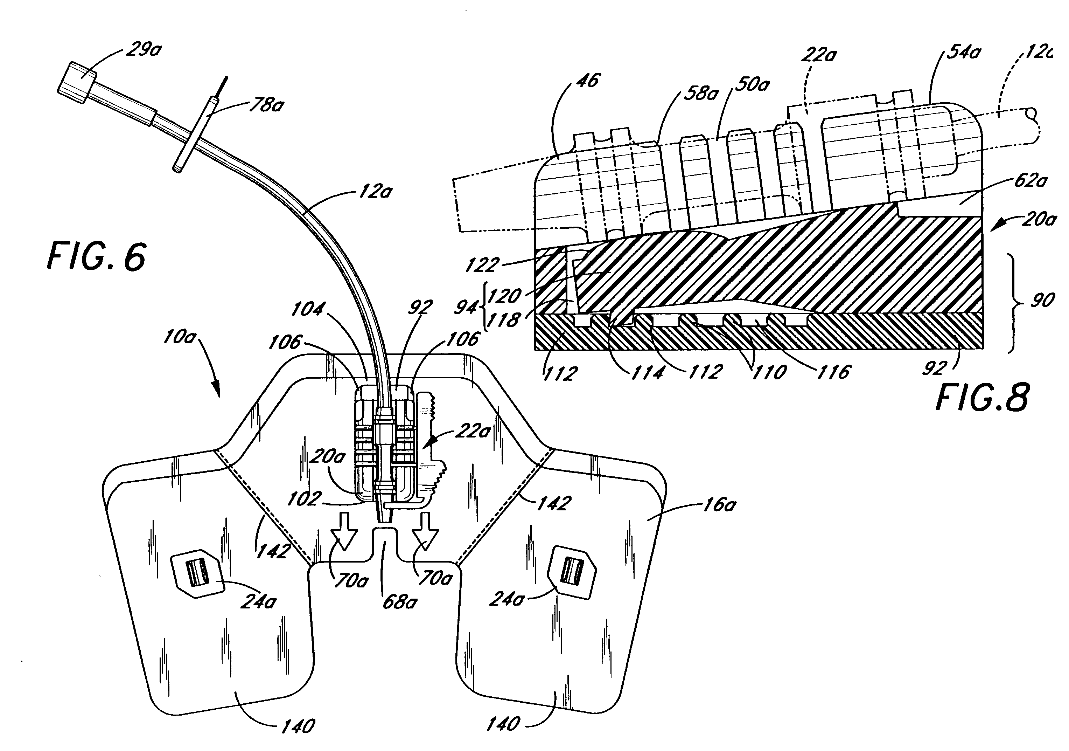

As best seen in Figure 8, the location adjustment mechanism 90 comprises a base 92 and interlocking mechanism 94 which interconnects the base 92 and the retainer 20a. The retainer 20a slides over the base 92 and the interlocking mechanism 94 secures the retainer 20a to the base 92 at various longitudinal positions. The adjustment mechanism thus allows for precise positioning of the retainer 20 relative to the catheter 14 after the pad 16 is attached to the patient's skin.

The base 92 has a generally parallelepiped shape and comprises a rail 96. Figure 7a best illustrates that the rail 96 desirably has a "dove-tail" configuration in cross section. That is, the rail 96 has a cross-sectional shape with a flat upper edge 98 and a pair of opposing side edges 100, each edge 100 being angled inward from the upper edge 98 toward the middle of the rail 96. The rail 96 extends along the longitudinal length of the base 92 from the distal end 102 of the base 92 to a point just short of the base proximal end 104. The base 92 includes a pair of stops 106 at the proximal end 104 which close off the proximal end of the rail 96.

An adhesive attaches the base 92 to base pad 16a. Alternatively, the base 92 may be attached to the base pad 16a by like means (e.g., embedding or otherwise weaving the base 92 into the base pad 16a) as well.

The retainer 20a, configured in accordance with the above-description, additionally comprises a groove 108 having a cross-sectional shape corresponding to that of the rail 96. The retainer groove 108 receives the base rail 96 in a manner permitting the retainer 20a to slide over the base 92, but preventing the retainer 20a from moving in the transverse direction away from the base 92. The base stops 106 also limit the retainer's longitudinal travel in a proximal direction.

The interlocking mechanism 94 comprises a plurality of teeth 110 disposed on an upper surface 112 of the base 92, and a pawl 114 connected to the retainer 20a. The teeth 110 desirably have generally rectangular cross-sectional shapes, and lie in seriatim along the longitudinal axis of the base 92. The upper edge of each tooth 110 includes a chamfer 112 to facilitate the engagement of the pawl 114 with a hollow 116 formed between adjacent teeth 110, as discussed below. The longitudinal length of each tooth 110 desirably extends generally normal to the longitudinal axis of the base 92.

The pawl 114 has a shape configured to insert into and engage with the hollow 116 defined between the teeth 110. The pawl 114 preferably has a width, measured in the longitudinal direction, slightly less than that of the hollow 116.

The retainer 20a comprises an aperture 118 extending between the retainer base surface 48a and the channel bottom surface 56a. A flexible finger 120 extends from the retainer 20a in a cantilever fashion and into the retainer aperture 118. The flexible finger 120 supports the pawl 114 at its distal end. Although Figure 8 illustrates the finger 120 as extending in the distal direction, it is contemplated that the finger 120 can alternatively extend in the proximal direction as well.

The flexible finger 120 preferably comprises a protuberance 122 which extends upwardly beyond the channel bottom surface 56a and into the central channel 44a with the finger 120 in an undeflected state. The cantilever nature of the finger 120 enables the finger 120 to deflect downward so that the protuberance 122 lies below the retainer bottom surface 56a. With the finger 120 so deflected, the pawl 114 engages the series of teeth 110. That is, the pawl 114 inserts into a hollow 116 defined between the teeth 110. The interengagement between pawl 114 and the teeth 110 prevents the retainer 20a from sliding over the base 92.

### S-Clip

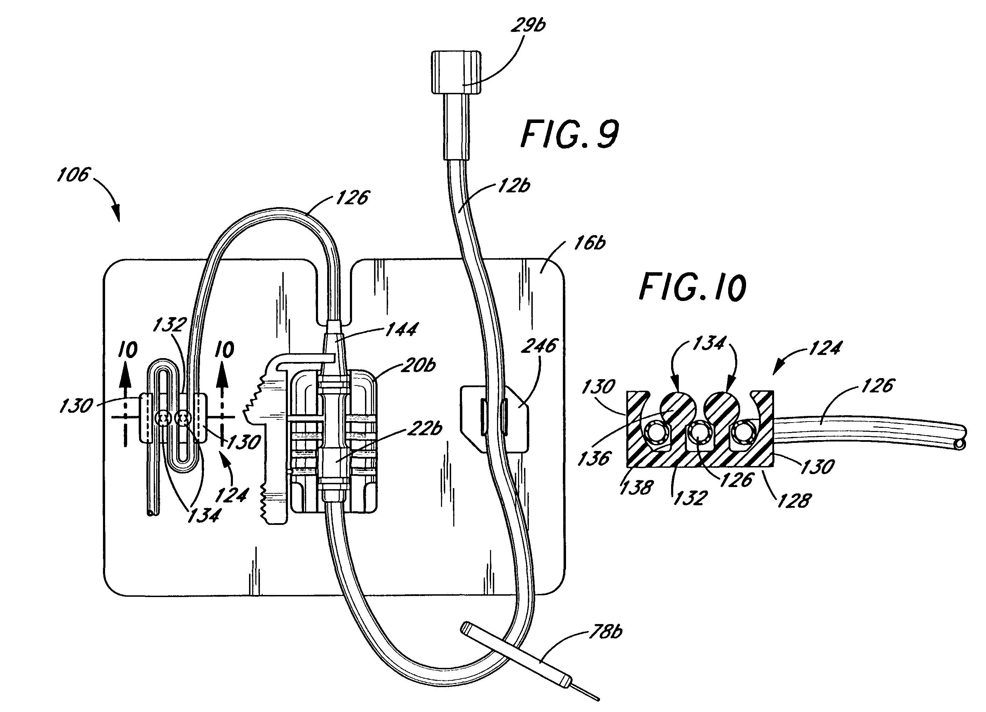

Figures 9 and 10 illustrate a catheter anchoring system 10b in accordance with a further embodiment of the present invention. Where appropriate, like numbers with an "b" suffix have been used to indicate like parts of the embodiments for ease of understanding.

The catheter anchoring system 10b is substantially identical to the anchoring system 10 first described above, with the addition of an S-clip 124 to retain a microbore or small bore tubing 126. The microbore tubing is commonly used, for example, with epidural catheterization procedures, as discussed in detail below.

The S-clip 124 comprises a generally U-shaped channel 128 defined by a pair of arcuate, upstanding walls 130 extending from a base plate 132. As best seen in Figure 10, the S-clip 124 further comprises a plurality of retainers 134, each retainer 134 having a spherical head 136 support by a cylindrical stem 138. The stems 138 extend from the base plate 132. The retainer stems 138 are positioned from one another and from the upstanding walls 130 by a distance slightly greater than the diameter of the microbore tubing 126. The retainers 134 are also positioned such that the spherical heads 136 of the retainers 134 are positioned from one another and from the upstanding walls 130 by a distance slightly less than the microbore tubing 126. As best seen in Figure 10, the retainer heads 136 prevent the microbore tubing 126 from disengaging from the S-clip 124 in the transverse direction once the microbore tubing 126 is snaked between the retainers 134 and the upstanding walls 130.

An adhesive attaches the base plate 132 of the S-clip 124 to base pad 16b. Alternatively, the base plate 132 may be attached to the base pad 16b by like means (e.g., embedding or otherwise weaving the base plate 132 into the base pad 16b) as well.

The components of the anchoring system 10, save the base pad 16 (i.e., the retainer 20, tube clip 24, adaptor 22, slide clamp 78, base 92 and S-clip 124), may be constructed in any of a variety of ways which will be well known to one of skill in the art. For instance, each individual component may be integrally molded such as by injection molding. The components preferably comprise a durably, flexible material, and more preferably comprise a generally inert, non-toxic material. In a preferred embodiment, the components are molded of plastic, such as, for example, polycarbonate, polyvinylchloride, polypropylene, polyurethane, tetrafluoroethylene (e.g., TEFLON®), polytetrafluoroethylene (a.k.a., PTFE), acetal resin (e.g., DELRIN®), chlorotrifluoroethylene (e.g., KEL-F®), nylon or like polymers.

### Method of Use

The following discussion of the method of use will be with reference to Figures 1 and 2, and initially will be in the context of intravenous catheterization. As the following discussion will illustrate, however, it is understood that the anchoring system 10 can be used in other catheterization procedures as well. The discussion of the method of use is intended to augment the above description of the invention, and, thus, should be read together.

A nurse typically begins the catheterization process by positioning the catheter 14 at a desired location above a vein. The nurse introduces a needle or other stylus through a cannula portion of the catheter 14 and into the skin of the patient at a desired angle of incident. For intravenous use, the catheter 14 commonly has an incident angle of approximately 7°. The nurse then inserts the cannula of the catheter 14 into the patient and withdraws the needle or stylus. The catheter hub 30 remains exposed above the skin.

The nurse inserts the distal end of the adaptor 26 into the catheter hub 30. The clip 34 has been slidably mounted in a most distal position so that it does not interfere with the insertion of the adaptor distal end 26 into the catheter hub 30.

The nurse then slides the clip 34 in a proximal direction to engage the catheter hub 30. In this manually selected position, the clip 34 securely attaches the adaptor 22 to the catheter 14. The rachet teeth of the adaptor 22 cooperate with the pawl to resist distal movement of the clip 34 and to hold the clip 34 in the manually selected position.

The nurse removes the paper backing which initially covers the adhesive bottom surface 18 of the base pad 16, and attaches the pad 16 to the patient's skin proximate to the indwelling catheter 14. Specifically, the nurse grips the backing tab 67 proximate to the retainer 20. The indicia 69 on the tab 67 indicates the location at which the nurse should grip the tab 67. The nurse then pulls on the tab 67 and peels the backing off one half of the bottom adhesive layer 18. The nurse positions the slot 68 of the pad 16 around the catheter cannula 14 with the instructing indicia 70 (e.g., indicating arrow) pointing in the direction of the catheter 14. The nurse then places the bottom layer 18 against the patient's skin to adhere the base pad 16 to the patient. Light pressure over the upper layer 64 assures good adhesion between the base pad 16 and the patient's skin. The base pad 16, due to its flexibility, conforms to the contours of the topical surface to which the base pad 16 adheres.

The nurse then repeats this procedure for the other half of the pad 16. Alternatively, the nurse may completely remove the backing from the pad 16 before attaching the pad 16 to the patient's skin.

The nurse orients the adaptor 22 with the clip 34 positioned to the side of the tubular body 25 (i.e., with the support arm 32 extending in the lateral direction) and locates the adaptor support arm 32 above the series of retainer slots 50 with the latch 36 positioned distal of the retainer distal end.

The nurse then snaps the adaptor 22 into the retainer 20 located proximal of the pad notch 68. In doing so, the adaptor 22 is pressed between the longitudinal walls 46 of the retainer 20 with the support arm 32 extending in a lateral direction. As the nurse presses the adaptor 22 into the retainer 20, the chamfered edges 58 around the slots 50 of the longitudinal wall 46 guide the support arm 32 into one of the slots 50.

As mentioned above, the opening 47 of the channel 46 has a smaller width measured in the lateral direction than the diameter of the tubular body 25. The lateral walls 46 thus deflect outwardly in a lateral direction. Once the tubular body 25 of the adaptor 22 rests within the central channel 44 of the retainer 20, the lateral walls 46 spring back to snap the adaptor 22 in place. The walls 46 of the retainer 20 thus prevent unintentional transverse and lateral movement of the adaptor 22.

In this position, the protuberance 40 of the adaptor 22 either rests either in a slot 50 or in the relief 60, proximal of the slot 50 through which the support arm 32 passes. The protuberance 40 engages a portion of the longitudinal wall 46, which forms either the relief 60 or the slot 50, to prevent the clip 34 from pivoting relative to the tubular body 25. The protuberance 40 thus ensures that the latch 36 maintains engagement with the catheter hub 30.

The slot 50 through which the support arm 32 passes prevents the adaptor 22 from sliding in the longitudinal direction. That is, the slot 50 prevents longitudinal displacement of the adaptor 22 when secured within the central channel 44.

The ergonomic design of the retainer 20 provides for a variety of positions of the adaptor 22 in the retainer 20 so that the retainer 22 is not technique or position sensitive. That is, a nurse can simply press the adaptor 22 into the retainer 20, irrespective of the side on which the support arm 32 is located, and irrespective of the position of the support arm 32 relative to a particular slot 50. So long as the support arm 32 is positioned above the series of slots 50, the chamfered edges 58 of the wall 46 will guide the support arm 32 into a slot 50. The protuberance 40 of the adaptor 22 also fits within an adjacent slot 50 or the relief 60.

With the support arm 32 extending through a slot 50 of the retainer 20, the adaptor 22 lies in a "low profile" position. That is, the support arm 32 of the adaptor 22 extends in the lateral direction to reduce the overall height of the anchoring system 10, as measured in the transverse direction. This position of the adaptor 22 reduces the risk of the system 10 interfering with surrounding action. The retainer 20, however, allows the adaptor 22 to rotate either to a position in which the support arm 32 extends in the transverse direction, or to a position 180° for the original position to locate the adaptor clip 34 on the opposite side of the retainer 20.

Once in the low profile position, the adaptor 22 will normally remain in this position until the adaptor 22 and its associated tubing 12 are removed and replaced by another.

As Figure 1 illustrates, the nurse may also form a safety loop in the fluid supply tubing 12, as known in the art, and secure the safety loop to the patient by inserting a portion of the tubing 12 into the tube clip 24. The safety loop absorbs any tension applied to the fluid supply tube to prevent the adaptor 22 and/or catheter 14 from being pulled.

A nurse may use the slide clamp 78 to remove the adaptor body 25 from the retainer 20. The nurse inserts the tab 82 of the slide clamp 78 into the key-way groove 62 on the proximal end of the retainer 20. Because the tab 82 has a larger width than the depth of the key-way groove 62, measured in the transverse direction, the tab 62 pries the tubular body 25 from the central channel 44 as the nurse inserts the tab 82 into the key-way groove 62 in the distal direction. The nurse may further use the slide clamp 78 to leverage the proximal end of the tubular body 25 out the upper opening 47 of the retainer 20. Having displaced the proximal end of the adaptor 22 from the retainer 20, the nurse may easily remove the adaptor distal end from of the retainer 20. Alternatively, the nurse may also remove the tubular body 25 by lifting up on the tubing 12 while holding down the pad 16 with the other hand.

Figures 6 through 8 illustrate the catheter anchoring system 10a particularly suited for arterial catheterization. Because of the criticality of the incident angle (i.e., the angle at which the catheter 14a projects into the patient) at which the catheter 14a must be maintained, it is advantageous to precisely position the retainer 20a so that the retainer 20a holds the catheter 14a at the desired incident angle. The desired range of incident angle commonly is about 5°-30° for arterial catheterization. The incident angle preferably ranges between about 15° and about 25°, and more preferably equals about 22°.

A nurse inserts the catheter cannula 14a into an artery in a similar manner to that described above in connection with intravenous catheterization. The nurse subsequently connects the adaptor 22a to the indwelling catheter 14a as previously described. The nurse also attaches the flexible pad 16a to the patient in a like manner to that described above. If desired, the nurse can remove one of the wings 140 of the pad 16a before attaching the pad 16a to the patient, by tearing the pad 16a along the perforation line 142.

The nurse orients the adaptor 22a with the clip 34a positioned to the side of the tubular body 25a (i.e., with the support arm 32a extending in the lateral direction) and locates the adaptor support arm 32a above the series of retainer slots 50a with the latch 36a positioned distal of the retainer distal end. If the nurse positions pad 16a too close to or too far from the indwelling catheter 14a, the nurse can slide the retainer 20a in the desired direction to locate the retainer slots 50a beneath the adaptor support arm 32a.

The nurse then snaps the adaptor 22a into the retainer 20a located proximal of the pad notch 68a. In doing so, the chamfered edges 58a around the slots 50a of the longitudinal wall 46a guide the support arm 32a into one of the slots 50a. The retainer 20a automatically slides longitudinally to precisely position a corresponding slot 50a beneath the support arm 32a. The adaptor 22a thus-snaps into the retainer 20a without causing the catheter 14a to move substantially.

The tubular body 25a contacts the protuberance 122 of the finger 120 and causes the finger 120 to deflect downward as the adaptor tubular body 25a snaps into the central channel 44a. In turn, the pawl 114 engages the series of teeth 110 which prevents longitudinal movement of the retainer 20a while holding the adaptor 20a. If the nurse removes the adaptor 22a -- preferably by using the slide clamp tab 82a -- the finger 120 springs back to its undeflected state and the retainer 20a freely slides over the rail 96. The pawl 114 normally does not engage the series of teeth 110.

The ability to precisely position the retainer 20a beneath the catheter adaptor 22a connected to the catheter 14a, enables the nurse to hold the catheter 14a in a stable position and ensures that the retainer 20a will hold the adaptor 22a, and thus the catheter 14a, at the precise incident angle. Without the ability to adjust the longitudinal position of the retainer 20a, the nurse may perform a series of position iterations before properly locating base pad 16a, and thus the retainer 20a, relative to the indwelling catheter 14a.

For epidural catheterization, an anesthesiologist, for example, inserts the distal end of microbore tubing 126 through the dura membrane and into the epidural space. The proximal end of the microbore tubing 126 conventionally includes a Toughy-Bourst adaptor 144 to couple with the fluid supply tube 12b transporting the anesthesia. It is imperative that the connection between the microbore tubing 126 and the fluid supply tubing 144 remain in tact, and that the distal end of the microbore tubing 126 remains in place. For if the dura membrane is exposed to air-borne microbes, meningitis may develop. Thus, a secure interconnection between the microbore tubing 126 and the fluid supply 12b should exist, and the microbore tubing 126 should be isolated from any tension placed on either the fluid supply tube 12b, as well as the adaptor 22b.

Figures 9 and 10 illustrate the catheter anchoring system 10b particularly suited for epidural catheterization. A doctor uses the present anchoring system 10b in a manner similar to that described above in connection with intravenous catheterization, with the exceptions that doctor connects the adaptor 22b to microbore tubing 126 and adheres the base pad 16b to the posterior of the patient's torso.

The doctor subsequently snakes the microbore tubing 126 through the S-clip 124 by first pressing the tubing 126 between a retainer 134 and the wall 130, and then wrapping the tubing 126 between the first and second retainers 134. Light pressure forces the tube 126 between the retainers 134. The doctor then wraps the tube 126 back between the second retainer 134 and the second wall 130, and presses the tube 126 therebetween. The S-clip 124 secures the microbore tube 126 in place and isolates the microbore tube 126 from tension placed on the adaptor 22b and/or the fluid supply tube 12b with the microbore tube 126 inserted accordingly.

### Additional Embodiments

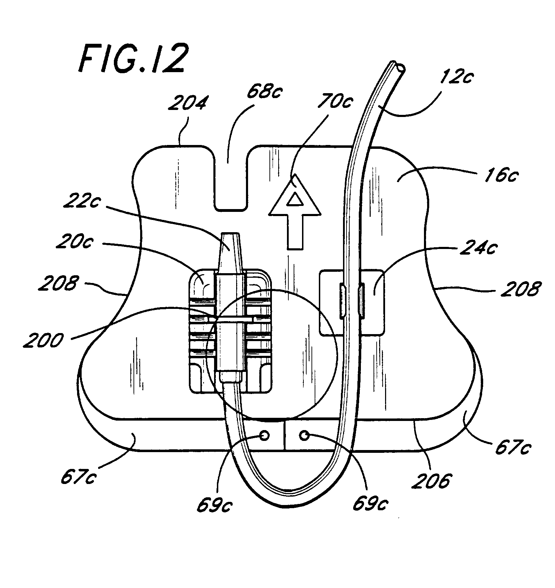

As mentioned above, it is contemplated that other types of adaptors in addition to the one disclosed above can be used as well with the present catheter anchoring system. Figures 11 and 12 illustrate a catheter anchoring system 10c in accordance with a further embodiment of the present invention which includes a different catheter adaptor style. Where appropriate, like numbers with a "c" suffix have been used to indicate like parts of the embodiments for ease of understanding.

Like the catheter anchoring systems described above, the present catheter anchoring system 10c principally comprises a flexible anchor pad 16c having an adhesive bottom side 18c, which attaches to the skin of the patient. The pad 16c supports a retainer 20c. The retainer 20c is configured to receive and secure in place a catheter adaptor 22c which connects to an indwelling catheter 14c. The pad 16a may also support a tube clip 24c which is used to retain a portion of the tubing 12c.

Figure 11 illustrates the adaptor 22c as comprising a generally tubular body 25c defined between a distal end 26c and a proximal end 28c. The proximal end 28c is adapted to receive a distal end of the tube 12c. In an exemplary embodiment, at least a portion of the fluid supply tube 12c is permanently attached to the body proximal end 28c.

The distal end 26c is configured to engage a proximal end of the indwelling catheter 14c (not shown). Although Figures 11 and 12 illustrate the distal end 26c of the adaptor 22c as having a frusto-conical shape configured to engage a standard lure-type catheter hub 30c (not shown), it is contemplated that the distal end 26c could be configured as well to engage other types of connectors.

Figure 14 illustrates an alternative configuration of the distal end 26d of the catheter adaptor 22d. Again, for consistency, like numbers with a "d" suffix have been used to indicate like parts of the catheter adaptor of Figure 11 and the catheter adaptor of Figure 14.

The catheter adaptor 22d includes a standard lure-lock type fitting 220 attached to the body 25d of the catheter adaptor 22d so as to circumscribe the distal end 26d of the catheter adaptor 22d. The lure-lock fitting 220 preferably is attached in a manner which permits the fitting 220 to be rotated about the catheter adaptor body 25d.

In the illustrated embodiment, the fitting 220 has a generally tubular shape with a closed proximal end 222. The closed end 222 includes an aperture 224 of a sufficient size to receive a part of the adaptor body 25d, as described below. The fitting 220 includes conventional internal threads 226 in order to engage corresponding threads of a conventional female lure-lock fitting (not shown).

The adaptor body 25d desirably includes an annular groove 228 which receives a portion of the closed end 222 of the fitting 220 to interconnect the fitting 220 and the adaptor body 25d. This interconnection also permits the fitting 220 to be rotated about the adaptor body 25d.

To assemble the catheter adaptor 22d, the conical shaped distal end 26d of the body 25d is inserted into the aperture 224 of fitting closed end 222. The body 25d is then forced into the fitting 220 to slightly deflect the closed end 222 until the closed end 222 snaps into the annular groove 228 of the body 25d. In this position, the body 25d captures a portion of the fitting 220 to couple these elements together.

With reference to Figure 11, the adaptor 22c includes an annular collar 200 interposed between the proximal and distal ends 28c, 26c of the tubular body 25c. The adaptor 22d of Figure 14 also includes a like annular collar 200d. It is contemplated that the collar 200 of the adaptor 22c of Figure 11 and the collar 200d of the adaptor 22d of Figure 14 will be substantially identical, and, thus, the description herein will be understood as applying equally to both embodiments.

The collar 200 is preferably positioned closer to the distal end 26c than the proximal end 28c. The distance between the distal end 26c and the collar 200 preferably equal approximately the distance between the distal end 26 and the support arm 32 of the above-described adaptor 22. In this manner, the present retainer 20c can be used with all of the above-described styles of catheter adaptors.

The annular collar 200 flares radially outwardly and circumscribes the tubular body 25c. The annular collar 200 has a thickness measured in a longitudinal direction which is slightly less than a width of a slot 50c in a retainer wall 46c so that the collar 200 fits within the slot 50c of a retainer wall 46c, as discussed in detail below.

The adaptor 22c is preferably formed of a durable, biocompatible plastic material. The adaptor 22c more preferably is formed of clear plastic so a nurse can see bubbles or backflow through the adaptor 22c. In an exemplary embodiment, the adaptor is formed of polycarbonate by injection molded; however, those skilled in the art will readily appreciate that the adaptor can be formed by other construction methods known in the art.

Figures 11 and 12 also illustrate the retainer 20c which is substantially identical to the retainer 20 described above. The retainer 20c comprises a central channel 44c interposed between a pair of opposing longitudinal walls 46c. The central channel 44c extends through the retainer 20c along an axis which is generally parallel to a longitudinal axis of the retainer 20c.

The central channel axis 44c has a generally circular cross-sectional shape which is truncated at an upper end to form an opening 47c. The central body 44c has a diameter sized to receive the tubular body 25c of the catheter adaptor 22c. In a preferred embodiment, the diameter of the central channel 44c generally matches that of the tubular body 25c.

In cross section, the central channel 44c extends through an arc greater than 180° about the channel axis such that the lateral length of the opening 47c is less than the diameter of the central channel 44c. In an exemplary embodiment, the cross-sectional shape of the central channel 44c extends through an arc of about 200° about the channel axis.

As best seen in Figure 13a, the channel axis is desirably skewed relative to a base surface 48c of the retainer 20c. An incident angle θ formed between the base surface 48c and the channel axis is less than 45°. The incident angle θ desirably ranges between 5° and 30°. In an exemplary embodiment for intravenous use, the angle θ preferably approximately equals 7°.

The longitudinal walls 46c are substantially identical. Each wall 46c has a thickness measured in the lateral direction less than the length of the support arm 32 of the adaptor 22, as it is desirable for the present retainer 20c to accept both the above-described adaptor 22 which comprises a support arm 32 connected to a clip 34, as well as the present adaptor 22c which comprises an annular collar 200. Preferably, the thickness of the wall 46c measured in the lateral direction is greater than the distance measured radially by which the collar 200 extends beyond the exterior surface of the tubular body 25c (i.e., a radial height). The length of each wall 46c, as measured in the longitudinal direction, is preferably coextensive with the length of the retainer 20c.

Each wall 46c comprises a uniform series of slot 50c. The series comprises at least two (2) slots 50c and not more than twenty (20) slots 50c. More preferably, the series comprises less than seven (7) slots 50c. In an exemplary embodiment, as illustrated in the figures, the series comprises four (4) slots 50c.

As discussed above, each slot 50c is sized to receive the collar 200 of the adaptor 22c, as well as the support arm 32 of the catheter adaptor 22, to prevent longitudinal displacement of the respective adaptor 22, 22c. Each slot 50c desirably has a rectangular shape. As seen in Figure 12, the slots 50c extend from an exterior surface 52c, through the wall 46c, and open into the central channel 44c. The width of the slot 50c, as measured in the longitudinal direction, is desirably slightly greater than the width of the support arm 32 and the width of the collar 200.

As illustrated by Figure 13a, each slot 50c extends in the transverse direction from an upper edge 54c of the longitudinal wall 46c to a point below the bottom 56c of the central channel 44c. The height of the slot 50c, as measured in the transverse direction, is thus greater than the distance between the upper edge 54c and the channel bottom 56c of the retainer 20c. As seen in Figure 13b, the retainer 20c further includes a series of lateral grooves 202 which extend between opposing slots 50c and extend into the retainer 20c from the channel bottom surface 56c. The opposing slots 50c and groove 202 thus form a lateral channel which extends through the retainer 20c in the lateral direction and cuts into the retainer 20c from the upper edge 54c to a point below the channel bottom surface 56c. The groove 202 desirably is sized to receive a portion of the collar 200 such that with the tubular body 25c positioned within the central channel 44c, the collar 200 extends between opposing slots 50c and into the groove 202. Thus, the groove 202 has a depth, measured between the lower surface 56c of the central channel 44c and the bottom of the groove in transverse direction, which is greater than the radial height of the annular collar 200.

Figure 13b illustrates that the spacing S between the slots 50c, on center, desirably equals about half the distal L (see Figure 2) between the support arm 32 and the protuberance 40 of the catheter adaptor 22. The position of the slots in relation to the proximal and distal ends 26c, 28c of the retainer 20c is desirably configured in accordance with the spacing and positioning discussed above in connection with the above-described retainer 22, such that the present retainer 22c can be used with the above-described adaptor 22, including a clip 34.

Figures 11 and 13a illustrate the upper edge of each longitudinal wall 46c which comprises a series of chamfers 58c formed and positioned as disclosed above in connection with the retainer 20. As discussed above, the chamfers 58c slope downwardly towards the slot 50c to facilitate the insertion of either the support arm 32 of the above-described catheter adaptor 22 or the annular collar 200 of the present catheter adaptor 22c into the slot 50c.

As Figures 11-13b illustrate, each longitudinal wall 46c may further comprise a relief 60c disposed on the proximal end of the retainer 20c. The configuration and position of the relief 60c desirably is in accordance with the above description of the retainer 20. Figure 11 further illustrates that the retainer 20c may additionally comprise a key-way groove 62c to facilitate removal of the catheter adaptor 22c from the retainer 20c, as discussed above. The key-way groove 62c desirably is also positioned and configured in accordance with the above disclosure in connection with the retainer 20.

The retainer 20c is made of relatively stiff plastic material, but is somewhat flexible such that the adaptor 22c forces the upper edges 54c of the longitudinal walls 46c outwardly when a nurse presses the adaptor 22c into the central channel 44c of the retainer 20c. The retainer 20c is desirably formed of polycarbonate by injection molding. When the adaptor 22c sits within the central channel 44c, the upper edges 54c of the walls 46c snap inwardly to their original position to securely hold the adaptor 22c within the retainer 20c.

An adhesive preferably attaches the retainer 20c to the anchor pad 16c. Alternatively, the retainer 20c may be attached to the anchor pad 16c by like means as well, e.g., embedding or otherwise weaving the retainer into the anchor pad 16c.

Figure 11 illustrates the anchor pad 16c as comprising a flexible, laminate structure comprising an upper paper or other woven or non-woven cloth layer 64c and a bottom adhesive layer 18c, with an inner cellulose foam layer 66c interposed therebetween. Alternatively, the flexible base pad 16 may comprise an adhesive bottom layer 18 and an upper cellulose form layer. An upper surface of the form layer is roughened by corona treating with a low electric charge, as known in the art. The foam layer 66c forms a cushion between the patient's skin and the rigid, plastic retainer 20c and tube clamp 24c. The adhesive layer 18c may comprise a coating of diaphoretic or nondiaphoretic material, depending upon the patient's skin condition. A medical grade foam tape with a diaphoretic adhesive is available commercially from NDM Manufacturers.

The removable paper or plastic backing (not shown) desirably covers the bottom adhesive layer 18 before use. As discussed above and illustrated in Figure 12, the backing is preferably divided into a plurality of piece and includes tabs 67c to ease removal of the backing from the pad 16. The tabs 67c include indicia 69c (e.g., dots, text, arrows, etc.) to indicate the location at which to grip the corresponding tab 67c when peeling the removable backing off the pad 16c.

As best seen in Figure 12, the anchor pad 16 desirably has a generally trapezoidal shape with rounded corners. A distal edge 206 of the anchor pad 16c desirably has a width, as measured in the lateral direction, wider than that of a proximal edge 204. The longer distal edge 206 provides a longer adhesive surface over a rough contact surface, such as, for example, over knuckles, vertebrae, or the like. The generally trapezoidal shape, however, minimizes the overall size of the anchor pad 16c attached to the patient. The trapezoidal shape also provides the same surface area as a square pad with a appearance of a smaller pad. The longitudinal sides 208 of the anchor pad 16c preferably taper from the proximal edge 206 to the distal edge 204, and more desirably have concave shapes.

The anchor pad includes a notch 68c positioned along the proximal edge 204 of the anchor pad 16c and adjacent to the point of insertion of the catheter cannula. Preferably, the notch 68c is symmetrically positioned about the channel axis 44c of the retainer 20c attached to the anchor pad 16c. The notch 68c is sized to permit visual inspection of the catheterized site and is large enough to allow for variable placement of the pad 16c with respect to the insertion site. That is, the notch 68c is large enough that a nurse is not required to precisely position the pad on the patient's skin with respect to the indwelling catheter 14c (not shown).

As seen in Figures 11 and 12, the anchor pad 16c desirably comprises indicia 70c sometimes in the form of an arrow which indicates the proper orientation of the anchor pad 16 in reference to the catheterized site. When properly used, the indicia 70c points toward the indwelling catheter 14c (not shown).

The anchor pad 16c preferably supports a clip 24c which secures the fluid supply tube 12c to the anchor pad 16c. As seen in Figure 12, the fluid supply tube 12c is preferably looped back around in a proximal direction and inserted into the clip 24c to form a safety loop, as known in the art. The tube clip 24c is desirably configured in accordance with the above description. The clip 24c may be made in a variety of sizes to accommodate various calibers of fluid flow tubing 12c.

In use, a nurse typically uses the catheter anchoring system 10c in connection with an indwelling catheter 14c (not shown). The catheter 14c is inserted into a body lumen, such as a vein, in accordance with the above description. The nurse then inserts the distal end 26c of the adaptor 22c into a catheter hub 30c (not shown) to connect the adaptor 22c to the catheter 14c.

The nurse removes the paper backing which initially covers the adhesive bottom surface 18c of the anchor pad 16c, as described above, and attaches the anchor pad 16c to the patient's skin proximate to the indwelling catheter 14c. The nurse specifically positions the notch 68c of the pad 16c around the catheter cannula 14c with the indicating arrow 70c pointing in the direction of the catheter 14c. The nurse generally aligns the proximal edge 204 of the anchor pad 16c with the insertion site.

The nurse positions the adaptor 22c above the series of retainer slots 50c, and snaps the adaptor 22c into the retainer 20c. In doing so, the adaptor 22c is pressed between the longitudinal walls 46c of the retainer 20c with the annular collar 200 extending into opposing slots 50c and into the corresponding groove 202 of the retainer 20c. As the nurse presses the adaptor into the retainer 20c, the chamfered edges 58c around the slots 50c of the longitudinal walls 46c guide the annular collar 200 into the slots 50c. The retainer 20c secures the adaptor 20c as described above.

With the annular collar 200 positioned in the opposing slots 50c the adaptor 22c is prevented from sliding in a longitudinal direction.

Like the above-described embodiments of the retainer, the ergonomic design of the retainer 20c provides for various positions of the adaptor 22c in the retainer 20c so that the retainer 22c is not technique- or position-sensitive. That is, a nurse can simply press the adaptor 22c into the retainer 20c, irrespective of the position of the annular collar 20 relative to a particular slot 50c of the retainer 20c. So long as the annular collar 200 is positioned above the series of slots 50c, the chamfered edges 58c of the wall 46c will guide the annular collar 200 into the slot 50c.

The present embodiment of the retainer 20c, as mentioned above, may also be used with the above-described adaptor 22 having the clip 34. A nurse uses the present retainer with the above-described adaptor 22 in the same manner as described above in connection with the above-described retainer 20.

If the catheter hub 30 (see Figure 1) is a standard female lure-lock fitting, the lure-lock fitting 220 (Figure 14) of the adaptor body 22d is rotated with the distal end 26d inserted into the catheter hub 30 to interlock the corresponding fittings 222, 30 in the known manner. The catheter adaptor 22d is then used with the anchoring system in a like manner to that described above.

Although this invention has been described in terms of certain preferred embodiments, other embodiments apparent to those of ordinary skill in the art are also within the scope of this invention. Accordingly, the scope of the invention is intended to be defined only by the claims which follow.

## Claims

1. An anchoring system comprising:
a catheter adaptor (22) defined between a distal end configured to permit engagement with tubing (126) or the proximal hub of a catheter or lure-type connector and a proximal end configured to couple to a distal end of a tube, the catheter adaptor (22) having a generally tubular body (25);
a retainer (20) having a longitudinal channel (44) configured to receive the catheter adaptor tubular body (25) in a snap fit manner;
a flexible anchor pad (16) including an adhesive bottom surface (18), the anchor pad (16) supporting the retainer (20); wherein
the catheter adaptor (22) further comprises a radially extending member (200) disposed along the length of the tubular body (25), which radially extending member projects from an exterior surface of the tubular body; wherein
the retainer (20) comprises a pair of opposed longitudinally extending walls (46), defining between them a longitudinal channel (44) having a generally circular cross section truncated at an upper end to form a generally U-shaped channel having an upper opening (47) and a series of slots (50), sized to receive and capture said radially extending member, the series of slots being formed in each of the walls (46), each slot in one wall being in alignment with a corresponding slot in the other wall.

2. The anchoring system of claim 1 wherein the longitudinal channel (44) has an axis which extends along the channel, and an angle is defined between the axis of the channel and a base surface of the retainer.

3. The anchoring system of claim 2 wherein the angle between the axis of the channel and the base surface of the retainer is less than 45°.

4. The anchoring system of claim 2 or claim 3 wherein the angle between the axis of the channel and the base surface of the retainer is between about 5 ° and 30°.

5. The anchoring system of any one of claims 1 to 4 wherein the series of slots comprises 4 slots.

6. The anchoring system of any one of claims 1 to 5 wherein the cross section of the channel extends about the axis of the channel through an arc of greater than 180°.

7. The anchoring system of any one of claims 1 to 6 wherein the cross section of the longitudinal channel remains substantially constant along the longitudinal length of the channel.

8. The anchoring system of any one of claims 1 to 7 wherein said radially extending member comprises a support arm (32) which connects a clip (34) to said tubular body (25).

9. The anchoring system of claim 8 wherein said clip (34) of said adaptor (22) is adapted to pivot relevant to said tubular body (25).

10. The anchoring system of any one of claims 1 to 9 wherein said radially extending member comprises an annular collar (200) which circumscribes said tubular body (25).

11. The anchoring system of any one of claims 1 to 10 wherein said catheter adaptor (22) additionally comprises a lure lock fitting positioned about said distal end of said tubular body (25).

12. The anchoring system of any one of claims 1 to 11 wherein the channel extends through said retainer about an axis which is generally parallel to said retainer longitudinal axis.

13. The anchoring system of any one of claims 1 to 12 wherein said retainer additionally comprises a key way groove (62) positioned on the proximal end of said retainer (20).

14. The anchoring system of any one of claims 1 to 13 wherein said radially extending member of said adaptor is a support arm (32) for a clip (34) attached to said adaptor body, said clip (34) including a protuberance (40), and wherein said slots (50) of said retainer (20) are spaced apart by a distance approximately equal to half of the distance between the support arm (32) and the protuberance (40) of the adaptor clip (34).

15. The anchoring system of any one of claims 1 to 14 additionally comprising a base (92) having a rail (96) extending along a longitudinal axis of said base (92) and wherein said retainer (20) comprises a groove (108) configured to receive said rail (96) of said base (92)) such that said retainer (20) slides over said base (92).

16. The anchoring system of claim 15 wherein said retainer groove (108) and said rail (96) are configured to prevent said retainer (20) from moving away from said rail (96) in a transverse direction.

17. The anchoring system of claim 16 additionally comprising an interlocking element (94) which prevents said retainer from sliding over said base (92) with the retainer (20) holding the catheter adaptor (22), said interlocking element (94) comprising a series of teeth (110) and a pawl (114) which engages said teeth (110).

18. The anchoring system of any one of claims 1 to 17 comprising a tube clip (34) configured to receive a portion of a fluid supply tube (12) attached to said catheter adaptor (22).

19. The anchoring system of any one of claims 1 to 18 additionally comprising an S-clip (124) having a plurality of retainers (134).

20. The anchoring system of any one of claims 1 to 19 wherein said retainer (134) comprises a spherical head (136) supported by a stem (138).

21. The anchoring system of any one of claims 1 to 20 wherein said anchor pad (16) comprises a cellulose foam layer and has a trapezoidal shape defined by parallel distal and proximal edges, said distal edge (206) having a length longer than that of said proximal edge (204), said proximal edge (204) comprising a notch (68c) to visually inspect a catheter cannula with said anchor pad (16) positioned thereabout.

22. The anchoring system of any one of claims 1 to 21 wherein said anchor pad (167) comprises indicia which identify a desired direction of orientation with respect to an indwelling catheter (140).

23. The anchoring system of any one of claims 1 to 22 wherein said anchor pad comprises rounded comers.

24. The anchoring system of any one of claims 1 to 23 wherein said anchor pad (16) comprises longitudinal side edges (208), each of said edges having a concave configuration.

25. The anchoring system of any one of claims 1 to 24 wherein said retainer (20) is attached to said anchor pad (16) at a position in which said notch (68c) generally aligns with said channel (44).

26. The anchoring system of any one of claims 1 to 25 wherein said anchor pad comprises a laminate structure formed by a cellulose foam layer interposed between an adhesive layer (18) and a layer of fibers.

27. The anchoring system of claim 26 wherein said foam layer has a roughened upper surface.

28. The anchoring system of any one of claims 1 to 27 wherein said anchor pad additionally comprises a paper backing removably covering said adhesive layer (18).

29. A method of anchoring an inter connection between a catheter adaptor and a tube (12; 12a; 12b; 12c; 12d) comprising:
a. providing a catheter adaptor (22) defined between a distal end configured to permit engagement with tubing (126) or the proximal hub of a catheter or lure-type connector and a proximal end configured to couple to a distal end of the tube, the catheter adaptor (22) having a generally tubular body (25);
b. providing a retainer (20) having a longitudinal channel (44) configured to receive in a snap fit manner the catheter adaptor tubular body (25);
c. providing a flexible anchor pad (16) including an adhesive bottom surface (18); the anchor pad (16) supporting the retainer (20); wherein the catheter adaptor (22) further comprises a radially extending member disposed along the length of the tubular body (25), which radially extending member (200) projects from an exterior surface of the tubular body; and
the retainer (20) comprises a pair of opposed longitudinally extending walls (46) defining between them a longitudinal channel (44) having a generally circular cross section truncated at an upper end to form a generally U-shaped channel having an upper opening 47 and a series of slots (50) sized to receive and capture said radially extending member, the series of slots being formed in each of the walls (46), each slot in one wall being in alignment with a corresponding slot in the other walls;
d. positioning the catheter adaptor radially extending portion above the series of slots (50); and
e. inserting the catheter adaptor tubular body (25) in to the longitudinal channel (44) and the catheter adaptor radially extending portion in one of the series of slots (50).

30. A method of anchoring according to claim 29 **characterised in that** the method comprises providing an anchor pad (16) with an adhesive back (18), the anchor pad (16) supporting the retainer (20).

31. A method of anchoring according to claim 29 or claim 30 **characterised in that** it includes the step of connecting the adaptor (22) to tubing (126).

32. An anchoring method according to any one of claims 29 to 31 **characterised in that** it comprises the further steps of:
inserting a distal end (26) of the adaptor (22) in to a proximal of a hub at the end of tubing (126);
engaging the hub with a clip which is slidably mounted on the adaptor (22);
sliding the clip proximally on the adaptor (22) to grasp the hub; and
locking the clip to maintain the clip in engagement with the hub.

33. An anchoring method according to claim 32 **characterised in that** it comprises the further steps of:
inserting a distal end (26) of the adaptor (22) into a proximal of a hub at the end of tubing (126), the hub being a female lure lock type fitting; and
rotating a corresponding lure-lock fitting of the adaptor (22) to engage the female lure-lock fitting.

34. The anchoring method of claim 33 **characterised in that** it comprises depressing a canti levered finger (120) of the retainer (20) with the adaptor (22) positioned within the retainer (20); and
engaging a pawl (114) with the series of teeth (110).

35. The anchoring method of claim 34 **characterised in that** the step of inserting the adaptor (22) into the retainer (20) causes the finger (120) to deflect.

36. The anchoring method claim 35 **characterised in that** the step of deflecting the finger (120) causes the pawl (114) to engage the teeth (110).

## Patentansprüche

1. Verankerungssystem, umfassend:
einen Katheteradapter (22), der zwischen einem entfernten Ende, welches konfiguriert ist, um Eingriff mit einem Schlauch (126) oder dem nahen Ansatzstück eines Katheters oder Verbindungsstück vom Angeltyp zuzulassen, und einem nahen Ende definiert ist, das zum Koppeln an ein entferntes Ende eines Schlauchs ausgelegt ist, wobei der Katheteradapter (22) einen allgemein röhrenförmigen Körper (25) aufweist;
eine Halteeinrichtung (20) mit einem Längskanal (44), der zum Aufnehmen des röhrenförmigen Katheteradapterkörpers (25) in einer Schnappsitzweise ausgelegt ist;
ein flexibles Ankerpolster (16), das eine untere Klebstofffläche (18) umfasst, wobei das Ankerpolster (16) die Halteeinrichtung (20) abstützt; wobei
der Katheteradapter (22) weiter ein sich radial erstreckendes Element (200) angeordnet entlang der Länge des röhrenförmigen Körpers (25) aufweist, welches sich radial erstreckende Element von einer Außenfläche des röhrenförmigen Körpers vorsteht, wobei
die Halteeinrichtung (20) ein Paar gegenüberliegender, sich in Längsrichtung erstreckender Wände (46) aufweist, die zwischen sich einen Längskanal (44) mit einem allgemein kreisförmigen Querschnitt begrenzen, welcher an einem oberen Ende zum Bilden eines allgemein U-förmigen Kanals mit einer oberen Öffnung (47) und einer Reihe von Schlitzen (50) gestutzt ist, die Abmessungen zum Aufnehmen und Einfangen des genannten sich radial erstreckenden Elements aufweisen, wobei die Reihe von Schlitzen in jeder der Wände (46) ausgebildet ist und jeder Schlitz in einer Wand mit einem entsprechenden Schlitz in der anderen Wand ausgerichtet ist.

2. Verankerungssystem nach Anspruch 1, bei dem der Längskanal (44) eine Achse aufweist, die sich den Kanal entlang erstreckt, und ein Winkel zwischen der Achse des Kanals und einer Grundfläche der Halteeinrichtung begrenzt wird.

3. Verankerungssystem nach Anspruch 2, bei dem der Winkel zwischen der Achse des Kanals und der Grundfläche der Halteeinrichtung weniger als 45° beträgt.

4. Verankerungssystem nach Anspruch 2 oder Anspruch 3, bei dem der Winkel zwischen der Achse des Kanals und der Grundfläche der Halteeinrichtung zwischen etwa 5° und 30° beträgt.

5. Verankerungssystem nach einem der Ansprüche 1 bis 4, bei dem die Reihe von Schlitzen 4 Schlitze umfasst.

6. Verankerungssystem nach einem der Ansprüche 1 bis 5, bei dem der Querschnitt des Kanals sich um die Achse des Kanals über einen Bogen von mehr als 180° erstreckt.

7. Verankerungssystem nach einem der Ansprüche 1 bis 6, bei dem der Querschnitt des Längskanals entlang der Längslänge des Kanals im wesentlichen konstant bleibt.

8. Verankerungssystem nach einem der Ansprüche 1 bis 7, bei dem das genannte sich radiale erstreckende Element einen Stützarm (32) aufweist, der eine Klammer (34) mit dem genannten röhrenförmigen Körper (25) verbindet.

9. Verankerungssystem nach Anspruch 8, bei dem die genannte Klammer (34) des genannten Adapters (22) ausgelegt ist, um relevant zu dem genannten röhrenförmigen Körper (25) zu schwenken.

10. Verankerungssystem nach einem der Ansprüche 1 bis 9, bei dem das genannte sich radial erstreckende Element einen ringförmigen Kragen (200) aufweist, der den genannten röhrenförmigen Körper (25) umschließt.

11. Verankerungssystem nach einem der Ansprüche 1 bis 10, bei dem der genannte Katheteradapter (22) zusätzlich ein Angelverschlussanschlussstück positioniert über dem genannten entfernten Ende des genannten röhrenförmigen Körper (25) aufweist.

12. Verankerungssystem nach einem der Ansprüche 1 bis 11, bei dem sich der Kanal durch die genannte Halteeinrichtung um eine Achse erstreckt, die allgemein parallel zu der genannten Längsachse der Halteeinrichtung ist.

13. Verankerungssystem nach einem der Ansprüche 1 bis 12, bei dem die genannte Halteeinrichtung zusätzlich eine Keilnut (62) positioniert am nahen Ende der genannten Halteeinrichtung (20) aufweist.

14. Verankerungssystem nach einem der Ansprüche 1 bis 13, bei dem das genannte sich radial erstreckende Element des genannten Adapters ein Stützarm (32) für eine Klammer (34) befestigt an dem genannten Adapterkörper ist; wobei die genannte Klammer (34) eine vorstehende Stelle (40) umfasst, und wobei die genannten Schlitze (50) der genannten Halteeinrichtung (20) um einen Abstand ungefähr gleich der Hälfte des Abstands zwischen dem Stützarm (32) und der vorstehenden Stelle (40) der Adapterklammer (34) beabstandet sind.

15. Verankerungssystem nach einem der Ansprüche 1 bis 14, das zusätzlich einen Grundteil (92) mit einer Schiene (96) aufweist, die sich entlang einer Längsachse des genannten Grundteils (92) erstreckt, und bei dem die genannte Halteeinrichtung (20) eine Nut (108) aufweist, die zum Aufnehmen der genannten Schiene (96) des genannten Grundteils (92) derart ausgelegt ist, dass die genannte Halteeinrichtung (20) über den genannten Grundteil (92) gleitet.

16. Verankerungssystem nach Anspruch 15, bei dem die genannte Halteeinrichtungsnut (108) und die genannte Schiene (96) ausgelegt sind, um Bewegung der genannten Halteeinrichtung (20) von der genannten Schiene (96) weg in einer Querrichtung zu verhindern.

17. Verankerungssystem nach Anspruch 16, das zusätzlich ein Verriegelungselement (94) aufweist, das Gleiten der genannten Halteeinrichtung über den genannten Grundteil (92) verhindert, wobei die genannte Halteeinrichtung (20) den Katheteradapter (22) hält; wobei das genannte Verriegelungselement (94) eine Reihe von Zähnen (110) und eine Klinke (114) aufweist, die mit den genannten Zähnen (110) ineinander greift.

18. Verankerungssystem nach einem der Ansprüche 1 bis 17 mit einer Rohrklammer (34), die zum Aufnehmen eines Teils eines an dem genannten Katheteradapter (22) befestigten Fluidzuführschlauchs (12) ausgelegt ist.

19. Verankerungssystem nach einem der Ansprüche 1 bis 18, das zusätzlich eine S-Klammer (124) mit einer Anzahl von Halteeinrichtungen (134) aufweist.

20. Verankerungssystem nach einem der Ansprüche 1 bis 19, bei dem die genannte Halteeinrichtung (134) einen kugelförmigen Kopf (136) gehalten durch einen Schaft (138) aufweist.

21. Verankerungssystem nach einem der Ansprüche 1 bis 20, bei dem das genannte Ankerpolster (16) eine Zelluloseschaumstoffschicht aufweist und eine durch parallele entfernte und nahe Kanten begrenzte trapezartige Form hat, wobei die genannte entfernte Kante (206) eine längere Länge als diejenige der genannten nahen Kante (204) aufweist, und die genannte nahe Kante (204) einen Ausschnitt (68c) zum visuellen Inspizieren einer Katheterkanüle mit dem genannten Ankerpolster (16) darum positioniert aufweist.

22. Verankerungssystem nach einem der Ansprüche 1 bis 21, bei dem das genannte Ankerpolster (167) Anzeigen aufweist, die eine gewünschte Ausrichtungsrichtung in Bezug zu einem Verweilkatheter (140) identifizieren.

23. Verankerungssystem nach einem der Ansprüche 1 bis 22, bei dem das genannte Ankerpolster abgerundete Ecken aufweist.

24. Verankerungssystem nach einem der Ansprüche 1 bis 23, bei dem das genannte Ankerpolster (16) Längsseitenkanten (208) aufweist, wobei jede der genannten Kanten einen konkaven Aufbau hat.

25. Verankerungssystem nach einem der Ansprüche 1 bis 24, bei dem die genannte Halteeinrichtung (20) an dem genannten Ankerpolster (16) an einer Position befestigt wird, in der der genannte Ausschnitt (68c) allgemein mit dem genannten Kanal (44) ausgerichtet ist.

26. Verankerungssystem nach einem der Ansprüche 1 bis 25, bei dem das genannte Ankerpolster eine Schichtstoffstruktur aufweist, die durch eine Zelluloseschaumstoffschicht eingefügt zwischen einer Klebstoffschicht (18) und einer Faserschicht gebildet wird.

27. Verankerungssystem nach Anspruch 26, bei dem die genannte Schaumstoffschicht eine angeraute obere Fläche aufweist.

28. Verankerungssystem nach einem der Ansprüche 1 bis 27, bei dem das genannte Ankerpolster zusätzlich eine Papierschutzfolie aufweist, die entfernbar die genannte Klebstoffschicht (18) bedeckt.

29. Verfahren zum Verankern einer Zwischenverbindung zwischen einem Katheteradapter und einem Schlauch (12; 12a, 12b; 12c; 12c; 12d), umfassend:
a. Vorsehen eines Katheteradapters (22), der zwischen einem entfernten Ende, welches konfiguriert ist, um Eingriff mit einem Schlauch (126) oder dem nahen Ansatzstück eines Katheters oder Verbindungsstück vom Angeltyp zuzulassen, und einem nahen Ende definiert ist, das zum Koppeln an ein entferntes Ende des Schlauchs ausgelegt ist, wobei der Katheteradapter (22) einen allgemein röhrenförmigen Körper (25) aufweist;
b. Vorsehen einer Halteeinrichtung (20) mit einem Längskanal (44), der zum Aufnehmen des röhrenförmigen Katheteradapterkörpers (25) in einer Schnappsitzweise ausgelegt ist;
c. Vorsehen eines flexiblen Ankerpolsters (16), das eine untere Klebstofffläche (18) umfasst; wobei das Ankerpolster (16) die Halteeinrichtung (20) hält; wobei der Katheteradapter (22) weiter ein sich radial erstreckendes Element angeordnet entlang der Länge des röhrenförmigen Körpers (25) aufweist, welches sich radial erstreckende Element (200) von einer Außenfläche des röhrenförmigen Körpers vorsteht; und die Halteeinrichtung (20) ein Paar gegenüberliegender, sich in Längsrichtung erstreckender Wände (46) aufweist, die zwischen sich einen Längskanal (44) mit einem allgemein kreisförmigen Querschnitt begrenzen, der an einem oberen Ende zum Bilden eines allgemein U-förmigen Kanals mit einer oberen Öffnung 47 und einer Reihe von Schlitzen (50) gestutzt ist, die Abmessungen zum Aufnehmen und Einfangen des genannten sich radial erstreckenden Elements haben, wobei die Reihe von Schlitzen in jeder der Wände (46) gebildet ist und jeder Schlitz in einer Wand mit einem entsprechenden Schlitz in den anderen Wänden ausgerichtet ist;
d. Positionieren des sich radial erstreckenden Teils des Katheteradapters über der Reihe von Schlitzen (50); und
e. Einführen des röhrenförmigen Katheteradapterkörpers (25) in den Längskanal (44) und des sich radial erstreckenden Teils des Katheteradapters in einen der Reihe von Schlitzen (50).

30. Verankerungsverfahren nach Anspruch 29, **dadurch gekennzeichnet, dass** das Verfahren umfasst, ein Ankerpolster (16) mit einer Klebstoffrückseite (18) zu versehen, wobei das Ankerpolster (16) die Halteeinrichtung (20) stützt.

31. Verankerungsverfahren nach Anspruch 29 oder Anspruch 30, **dadurch gekennzeichnet, dass** es den Schritt zum Verbinden des Adapters (22) mit dem Schlauch (126) umfasst.

32. Verankerungsverfahren nach Anspruch 29 oder 31, **dadurch gekennzeichnet, dass** es die weiteren Schritte aufweist:
Einführen eines entfernten Endes (26) des Adapters (22) in einen nahen Teil eines Ansatzstücks am Ende des Schlauchs (126);
Ergreifen des Ansatzstücks mit einer Klammer, die verschiebbar an dem Adapter (22) angebracht ist;
Verschieben der Klammer nahe an dem Adapter (22) zum Ergreifen des Ansatzstücks; und
Verriegeln der Klammer zum Halten der Klammer in Eingriff mit dem Ansatzstück.

33. Verankerungsverfahren nach Anspruch 32, **dadurch gekennzeichnet, dass** es die weiteren Schritt umfasst:
Einführen eines entfernten Endes (26) des Adapters (22) in einen nahen Teil eines Ansatzstücks am Ende des Schlauchs (126), wobei das Ansatzstück ein Hohlanschlussstück vom Angelverschlusstyp ist; und
Drehen eines entsprechenden Angelverschlussanschlussstücks des Adapters (22) zum Eingreifen in das Hohlangelverschlussanschlussstück.

34. Verankerungsverfahren nach Anspruch 33, **dadurch gekennzeichnet, dass** es umfasst, einen ausgekragten Finger (120) der Halteeinrichtung (20) niederzudrücken, wenn der Adapter (22) innerhalb der Halteeinrichtung (20) positioniert ist; und
eine Klinke (114) mit einer Reihe von Zähnen (110) zu ergreifen.

35. Verankerungsverfahren nach Anspruch 34, **dadurch gekennzeichnet, dass** der Schritt zum Einführen des Adapters (22) in die Halteeinrichtung (20) Verbiegung des Fingers (120) verursacht.

36. Verankerungsverfahren nach Anspruch 35, **dadurch gekennzeichnet, dass** der Schritt zum Verbiegen des Fingers (120) die Klinke (114) veranlasst, die Zähne (110) zu ergreifen.

## Revendications

1. Système d'ancrage comprenant :
un adaptateur de cathéter (22) défini entre une extrémité distale configurée pour permettre l'engagement avec un tuyau (126) ou le centre proche d'un cathéter ou d'un connecteur de type leurre et une extrémité proche configurée pour se coupler à une extrémité distale d'un tube, l'adaptateur de cathéter (22) ayant un corps généralement tubulaire (25) ;
une retenue (20) ayant une gorge longitudinale (44) configurée pour recevoir le corps tubulaire (25) de l'adaptateur de cathéter en un engagement par encliquetage ;
un patin d'ancrage souple (16) comportant une surface inférieure adhésive (18), le patin d'ancrage (16) supportant la retenue (20) ; dans lequel
l'adaptateur de cathéter (22) comprend en outre un élément d'extension radiale (200) disposé sur la longueur du corps tubulaire (25), lequel élément d'extension radiale dépasse d'une surface extérieure du corps tubulaire; dans lequel
la retenue (20) comprend une paire de parois opposées d'extension longitudinale (46), définissant entre elles une gorge longitudinale (44) ayant une coupe transversale généralement circulaire tronquée à une extrémité supérieure afin de former une gorge en forme générale de U ayant une ouverture supérieure (47) et une pluralité de fentes (50), dimensionnées pour recevoir et capturer ledit élément d'extension radiale, la série de fentes étant formée dans chacune des parois (46), chaque fente dans une paroi étant en alignement avec une fente correspondante dans l'autre paroi.

2. Système d'ancrage selon la revendication 1, dans lequel la gorge longitudinale (44) a un axe qui s'étend le long de la gorge, et un angle est défini entre l'axe de la gorge et une surface de base de la retenue.

3. Système d'ancrage selon la revendication 2, dans lequel l'angle entre l'axe de la gorge et la surface de base de la retenue est inférieur à 45°.

4. Système d'ancrage selon la revendication 2 ou la revendication 3, dans lequel l'angle entre l'axe de la gorge et la surface de base de la retenue est environ entre 5° et 30°.

5. Système d'ancrage selon l'une quelconque des revendications 1 à 4, dans lequel la série de fentes comprend 4 fentes.

6. Système d'ancrage selon l'une quelconque des revendications 1 à 5, dans lequel la coupe transversale de la gorge s'étend autour de l'axe de la gorge sur un arc de plus de 180°.

7. Système d'ancrage selon l'une quelconque des revendications 1 à 6, dans lequel la coupe transversale de la gorge longitudinale reste substantiellement constante sur la longueur longitudinale de la gorge.

8. Système d'ancrage selon l'une quelconque des revendications 1 à 7, dans lequel ledit élément d'extension radiale comprend un bras de support (32) qui connecte un clip (34) audit corps tubulaire (25).

9. Système d'ancrage selon la revendication 8, dans lequel ledit clip (34) dudit adaptateur (22) est adapté pour pivoter par rapport audit corps tubulaire (25).

10. Système d'ancrage selon l'une quelconque des revendications 1 à 9, dans lequel ledit élément d'extension radiale comprend un collier annulaire (200) qui circonscrit ledit corps tubulaire (25).

11. Système d'ancrage selon l'une quelconque des revendications 1 à 10, dans lequel ledit adaptateur de cathéter (22) comprend en outre un raccord de verrouillage de leurre positionné autour de ladite extrémité distale dudit corps tubulaire (25).

12. Système d'ancrage selon l'une quelconque des revendications 1 à 11, dans lequel la gorge s'étend à travers ladite retenue autour d'un axe qui est généralement parallèle à l'axe longitudinal de ladite retenue.

13. Système d'ancrage selon l'une quelconque des revendications 1 à 12, dans lequel ladite retenue comprend de plus une rainure de, clavette (62) positionnée sur l'extrémité proche de ladite retenue (20).

14. Système d'ancrage selon l'une quelconque des revendications 1 à 13, dans lequel ledit élément d'extension radiale dudit adaptateur est un bras de support (32) pour un clip (34) attaché audit corps d'adaptateur, ledit clip (34) comportant une protubérance (40), et dans lequel lesdites fentes (50) de ladite retenue (20) sont espacées par une distance approximativement égale à la moitié de la distance entre le bras de support (32) et la protubérance (40) du clip d'adaptateur (34).

15. Système d'ancrage selon l'une quelconque des revendications 1 à 14, comprenant de plus une base (92) ayant un rail (96) s'étendant le long d'un axe longitudinal de ladite base (92) et dans lequel ladite retenue (20) comprend une rainure (108) configurée pour recevoir ledit rail (96) de ladite base (92) de telle sorte que ladite retenue (20) glisse par-dessus ladite base (92).

16. Système d'ancrage selon la revendication 15, dans lequel ladite rainure de retenue (108) et ledit rail (96) sont configurés pour empêcher ladite retenue (20) de s'écarter dudit rail (96) dans un sens transversal.

17. Système d'ancrage selon la revendication 16, comprenant de plus un élément d'interverrouillage (94) qui empêche ladite retenue de glisser par-dessus ladite base (92) avec la retenue (20) maintenant l'adaptateur de cathéter (22), ledit élément d'interverrouillage (94) comprenant une série de dents (110) et un cliquet (114) qui engage lesdites dents (110).

18. Système d'ancrage selon l'une quelconque des revendications 1 à 17, comprenant un clip de tube (34) configuré pour recevoir une partie d'un tube d'alimentation de fluide (12) attaché audit adaptateur de cathéter (22).

19. Système d'ancrage selon l'une quelconque des revendications 1 à 18, comprenant de plus un clip en S (124) ayant une pluralité de retenues (134).

20. Système d'ancrage selon l'une quelconque des revendications 1 à 19, dans lequel ladite retenue (134) comprend une tête sphérique (136) supportée par une tige (138).

21. Système d'ancrage selon l'une quelconque des revendications 1 à 20, dans lequel ledit patin d'ancrage (16) comprend une couche de mousse de cellulose et a une forme trapézoïdale définie par des bords distal et proche parallèles, ledit bord distal (206) ayant une longueur supérieure à celle dudit bord proche (204), ledit bord proche (204) comprenant une encoche (68c) pour inspecter visuellement une canule de cathéter avec ledit patin d'ancrage (16) positionné autour.

22. Système d'ancrage selon l'une quelconque des revendications 1 à 21, dans lequel ledit patin d'ancrage (167) comprend des marques qui identifient un sens d'orientation désiré par rapport à un cathéter fixe (140).

23. Système d'ancrage selon l'une quelconque des revendications 1 à 22, dans lequel ledit patin d'ancrage comprend des coins arrondis.

24. Système d'ancrage selon l'une quelconque des revendications 1 à 23, dans lequel ledit patin d'ancrage (16) comprend des bords latéraux longitudinaux (208), chacun desdits bords ayant une configuration concave.

25. Système d'ancrage selon l'une quelconque des revendications 1 à 24, dans lequel ladite retenue (20) est attachée audit patin d'ancrage (16) à une position à laquelle ladite encoche (68c) s'aligne généralement avec ladite gorge (44).

26. Système d'ancrage selon l'une quelconque des revendications 1 à 25, dans lequel ledit patin d'ancrage comprend une structure feuilletée formée d'une couche de mousse de cellulose interposée entre une couche adhésive (18) et une couche de fibres.

27. Système d'ancrage selon la revendication 26, dans lequel ladite couche de mousse a une surface supérieure rugueuse.

28. Système d'ancrage selon l'une quelconque des revendications 1 à 27, dans lequel ledit patin d'ancrage comprend en outre un revers en papier couvrant de manière amovible ladite couche adhésive (18).

29. Procédé d'ancrage d'une interconnexion entre un adaptateur de cathéter et un tube (12 ; 12a ; 12b ; 12c ; 12d) comprenant :
a. la fourniture d'un adaptateur de cathéter (22) défini entre une extrémité distale configurée pour permettre l'engagement avec un tube (126) ou le centre proche d'un cathéter ou d'un connecteur de type leurre et une extrémité proche configurée pour se coupler à une extrémité distale du tube, l'adaptateur de cathéter (22) ayant un corps généralement tubulaire (25);
b. la fourniture d'une retenue (20) ayant une gorge longitudinale (44) configurée pour recevoir en un engagement par encliquetage le corps tubulaire (25) de l'adaptateur de cathéter ;
c. la fourniture d'un patin d'ancrage souple (16) comportant une surface inférieure adhésive (18) ; le patin d'ancrage (16) supportant la retenue (20) ; dans lequel l'adaptateur de cathéter (22) comprend en outre un élément d'extension radiale disposé sur la longueur du corps tubulaire (25), lequel élément d'extension radiale (200) dépasse d'une surface extérieure du corps tubulaire ; et la retenue (20) comprend une paire de parois opposées d'extension longitudinale (46) définissant entre elles une gorge longitudinale (44) ayant une coupe transversale généralement circulaire tronquée à une extrémité supérieure afin de former une gorge en forme générale de U ayant une ouverture supérieure 47 et une pluralité de fentes (50) dimensionnées pour recevoir et capturer ledit élément d'extension radiale, la série de fentes étant formée dans chacune des parois (46), chaque fente dans une paroi étant en alignement avec une fente correspondante dans les autres parois.
d. le positionnement de la partie d'extension radiale de l'adaptateur de cathéter par-dessus la série de fentes (50) ; et
e. l'insertion du corps tubulaire (25) de l'adaptateur de cathéter dans la gorge longitudinale (44) et de la partie d'extension radiale de l'adaptateur de cathéter dans l'une de la série de fentes (50).

30. Procédé d'ancrage selon la revendication 29, **caractérisé en ce que** le procédé comprend la fourniture d'un patin d'ancrage (16) avec un revers adhésif (18), le patin d'ancrage (61) supportant la retenue (20).

31. Procédé d'ancrage selon la revendication 29 ou la revendication 30, **caractérisé en ce qu'**il comporte l'étape de connexion de l'adaptateur (22) au tube (126).

32. Procédé d'ancrage selon l'une quelconque des revendications 29 à 31, **caractérisé en ce qu'**il comprend les autres étapes :
d'insertion d'une extrémité distale (26) de l'adaptateur (22) dans une partie proche d'un centre à l'extrémité du tube (126) ;
d'engagement du centre avec un clip qui est monté de manière coulissante sur l'adaptateur (22) ;
de glissage du clip sur une partie proximale de l'adaptateur (22) afin de serrer le centre ; et
de verrouillage du clip afin de maintenir le clip en engagement avec le centre.

33. Procédé d'ancrage selon la revendication 32, **caractérisé en ce qu'**il comprend les autres étapes :
d'insertion d'une extrémité distale (26) de l'adaptateur (22) dans une partie proche d'un centre à l'extrémité du tube (126), le centre étant un raccord du type à verrouillage à leurre femelle ; et
de rotation d'un raccord de verrouillage de leurre correspondant de l'adaptateur (22) afin d'engager le raccord de verrouillage de leurre femelle.

34. Procédé d'ancrage selon la revendication 33, **caractérisé en ce qu'**il comprend l'enfoncement d'un doigt en porte à faux (120) de la retenue (20) avec l'adaptateur (22) positionné au sein de la retenue (20) ; et
l'engagement d'un cliquet (114) avec la série de dents (110).

35. Procédé d'ancrage selon la revendication 34, **caractérisé en ce** l'étape d'insertion de l'adaptateur (22) dans la retenue (20) entraîne un fléchissement du doigt (120).

36. Procédé d'ancrage selon la revendication 35, **caractérisé en ce** l'étape de fléchissement du doigt (120) entraîne l'engagement du cliquet (114) avec les dents (110).
